# EUROPEAN PATENT APPLICATION

(11) **EP 4 082 578 A1**
(43) Date of publication of application: **02.11.2022**
(21) Application number: 20904573.1
(22) Date of filing: 25.12.2020
(51) Int. Cl.: A61K 47/20, A61K 31/5377, A61P 35/00, G01N 23/2055, G01N 24/08, G01N 30/02, G01N 30/88, G01N 33/15

(54) **METHOD FOR SELECTING, ASSESSING, OR PRODUCING SODIUM LAURYL SULFATE AS RAW MATERIAL FOR PHARMACEUTICAL FORMULATION, OR FORMULATION OR THE LIKE CONTAINING SAME**

(30) Priority: 27.12.2019 JP 2019239018
(71) Applicant: CHUGAI SEIYAKU KABUSHIKI KAISHA, Tokyo 115-8543 (JP)
(72) Inventor: NAKAYAMA Tadanobu, Tokyo 115-8543 (JP); KURE Yoshiko, Tokyo 115-8543 (JP); SASAYAMA Takuro, Fujieda-shi, Shizuoka 426-0041 (JP)
(74) Representative: Vossius & Partner Patentanwälte Rechtsanwälte mbB
(86) International application number: PCT/JP2020/048639
(87) International publication number: WO 2021/132541

(57) **Abstract**

The present invention provides a method for determining a sodium lauryl sulfate having a desired quality as a pharmaceutical raw material for a formulation by detecting a slight difference in quality of sodium lauryl sulfate to be used as a pharmaceutical raw material for a formulation.

The present invention relates to a method for sorting a raw material for a formulation which can provide a pharmaceutical formulation excellent in stability by pretreating the material for a formulation under a predetermined accelerated condition and detecting an impurity. According to the method of the present invention, it is possible to determine the quality of sodium lauryl sulfate of a pharmaceutical raw material for a formulation having no effect on the quality of a pharmaceutical formulation containing alectinib or a salt thereof. A pharmaceutical composition produced by using a pharmaceutical raw material for a formulation (SLS) and sorted out by the method of the present invention can provide a high-quality pharmaceutical formulation having excellent stability.

## Description

### Technical Field

The present invention relates to, in a pharmaceutical formulation containing sodium lauryl sulfate, a method for sorting sodium lauryl sulfate of a pharmaceutical raw material for a formulation, a method for evaluating the quality of a pharmaceutical formulation containing the pharmaceutical raw material for a formulation, and a method for manufacturing a highly stable pharmaceutical formulation using sorted sodium lauryl sulfate. The present invention also relates to a sorted pharmaceutical raw material for a formulation, a pharmaceutical composition or pharmaceutical formulation containing a predetermined amount of the sorted pharmaceutical raw material for a formulation and/or its impurity.

### Background Art

A compound represented by formula (I) (compound name: 9-ethyl-6,6-dimethyl-8-(4-morpholin-4-yl-piperidin-1-yl)-11-oxo-6,11-dihydro-5H-benzo[b]carbazole-3-carbonitrile, substance name: alectinib): 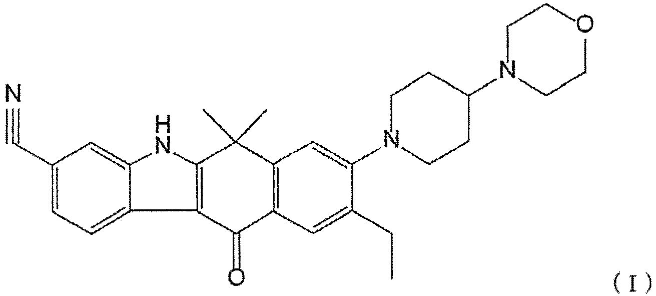 has an anaplastic lymphoma kinase (ALK) inhibitory activity. An alectinib hydrochloride is used as a therapeutic drug for ALK fusion-gene positive unresectable, advanced/recurrent non-small-cell lung cancer (Non Patent Literature 1).

Alectinib is a poorly soluble basic compound. Therefore, for formulation of alectinib, it has been reported that a composition of alectinib is prepared in the presence of a dissolution aid (Patent Literature 1); and that a capsule of alectinib having good elution profile is prepared by forming granules containing a compound represented by formula (I) or a salt thereof and blending the granules together with a disintegrant.

Sodium lauryl sulfate is used as an additive for various pharmaceutical formulations including alectinib formulations (Non Patent Literature 1). It is known that a formulation containing sodium lauryl sulfate is poor in stability, and that a formulation containing sodium lauryl sulfate poor in quality varies in characteristics. In order to stabilize a sodium lauryl sulfate-containing formulation, blending at least one selected from the group consisting of a neutral salt and a basic substance is known (Patent Literature 3).

However, it has never been known that if the quality of sodium lauryl sulfate of a pharmaceutical raw material for a formulation slightly changes, the quality of the resultant formulation is affected and deteriorates. Accordingly, a method for detecting a slight difference in quality of sodium lauryl sulfate has not been known.

### Citation List

### Patent Literatures

Patent Literature 1: Japanese Patent No. 4918630
Patent Literature 2: Japanese Patent No. 5859712
Patent Literature 3: Japanese Patent No. 4902928

### Non Patent Literature

Non Patent Literature 1: Package insert of ALECENSA 150-mg capsule, revised in December, 2018

### Summary of Invention

### Technical Problem

It has been desired to develop a method for detecting a slight difference in quality of sodium lauryl sulfate of a raw material for manufacturing a pharmaceutical formulation, thereby determining sodium lauryl sulfate having a desired quality as a raw material for manufacturing a pharmaceutical formulation.

### Solution to Problem

The present inventors repeatedly conducted intensive studies in consideration of the above problem. As a result, they found that a slight difference in quality of sodium lauryl sulfate contained in a formulation containing alectinib or a salt thereof influences stability and dissolution of the formulation; and that the above desire can be attained by the following method including a pretreatment of sodium lauryl sulfate under an accelerated condition. Based on the findings, the present invention was accomplished.

More specifically, the present invention relates to the following methods for sorting a pharmaceutical raw material for a formulation consisting essentially of sodium lauryl sulfate.
<1> A method for sorting a pharmaceutical raw material for a formulation consisting essentially of sodium lauryl sulfate, , the method comprising detecting an impurity after pretreating the pharmaceutical raw material for a formulation under an accelerated condition.
<2> The sorting method according to <1>, wherein the accelerated condition is a temperature condition of 65 to 75°C.
<3> The sorting method according to <1> or <2>, wherein the accelerated condition is a humidity condition of less than 79%RH.
<4> The sorting method according to any one of <1> to <3>, wherein the accelerated condition is a humidity condition of about 10%RH.
<5> The sorting method according to any one of <1> to <4>, wherein the accelerated condition includes a temperature condition of about 70°C and a humidity condition of about 10%RH or less.
<6> The sorting method according to any one of <1> to <3>, wherein the impurity is a sulfate ion, dodecanol or a sodium lauryl sulfate-dodecanol complex.
<7> The sorting method according to any one of <1> to <6>, wherein the impurity is detected by X-ray powder diffraction, ion exchange chromatography, gas chromatography or quantitative NMR.
<8> The sorting method according to any one of <1> to <7>, wherein the impurity is a sulfate ion, and the impurity is detected by ion exchange chromatography.
<9> The sorting method according to any one of <1> to <8>, wherein the pretreatment is performed under the accelerated condition for 2 days or more.
<10> The sorting method according to any one of <1> to <8>, wherein the pretreatment is performed under the accelerated condition for 4 days.
<10a> The sorting method according to any one of <1> to <10>, wherein the impurity is a sulfate ion, and a pharmaceutical raw material is sorted out in which the content of the sulfate ion is 1 mg/L or less.
<10b> The sorting method according to any one of<1> to <10a>, wherein the impurity is a sulfate ion, and a pharmaceutical raw material is sorted out in which the content of the sulfate ion is 1 mg/L or less in a solution prepared by dissolving about 100 mg of the pharmaceutical raw material for a formulation in water of 1 L.

Another embodiment of the present invention is a method for evaluating the quality of a pharmaceutical formulation of alectinib containing sodium lauryl sulfate of a pharmaceutical raw material for a formulation, by skipping measuring an impurity of the pharmaceutical formulation in case where the formulation contains the raw material which was sorted out by detecting an impurity under an accelerated condition.
<2> A method for evaluating the quality of a pharmaceutical formulation containing a compound represented by formula (I) 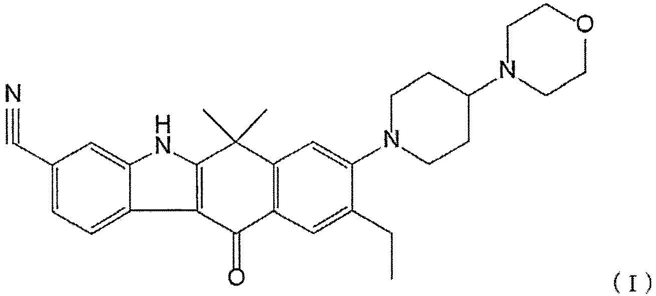 or a salt thereof and a pharmaceutical raw material for a formulation consisting essentially of sodium lauryl sulfate, the method comprising:
   a) selecting a pharmaceutical formulation containing a sorted pharmaceutical raw material for a formulation, wherein the sorted pharmaceutical raw material for a formulation is the pharmaceutical raw material for a formulation which was sorted out by detecting an impurity after pretreating the pharmaceutical raw material for a formulation under the accelerated condition; and
   b) skipping impurity measurement of the pharmaceutical formulation selected in step a.
<2a> The quality evaluation method according to <2>, wherein the accelerated condition is a temperature condition of 65 to 75°C.
<2b> The quality evaluation method according to <2> or <2a>, wherein the accelerated condition is a humidity condition of less than 79%RH.
<2c> The quality evaluation method according to any one of <2> to <2b>, wherein the accelerated condition is a humidity condition of about 10%RH or less.
<2d> The quality evaluation method according to any one of <2> to <2c>, wherein the accelerated condition includes a temperature condition of about 70°C and a humidity condition of about 10%RH or less.
<2e> The quality evaluation method according to any one of <2> to <2d>, wherein the impurity is a sulfate ion, dodecanol or a sodium lauryl sulfate-dodecanol complex.
<2f> The quality evaluation method according to any one of <2> to <2e>, wherein the impurity is detected by X-ray powder diffraction, ion exchange chromatography, gas chromatography or quantitative NMR.
<2g> The quality evaluation method according to any one of <2> to <2f>, wherein the impurity is a sulfate ion, and the impurity is detected by ion exchange chromatography.
<2h> The quality evaluation method according to any one of <2> to <2g>, wherein the pretreatment is performed under the accelerated condition for 2 days or more.
<2i> The evaluating method according to any one of <2> to <2h>, wherein the pretreatment is performed under the accelerated condition for 4 days.
<2j> The quality evaluation method according to any one of <2> to <2i>, wherein the impurity is a sulfate ion, and a pharmaceutical formulation is sorted out in which the content of the sulfate ion is 1 mg/L or less.
<2k> The quality evaluation method according to any one of <2> to <2j>, wherein the impurity is a sulfate ion, and a pharmaceutical formulation is sorted out in which the content of the sulfate ion is 1 mg/L or less in a solution prepared by dissolving about 100 mg of the pharmaceutical raw material for a formulation in water of 1 L.

Another embodiment of the present invention relates to a method for sorting a pharmaceutical raw material for a formulation (sodium lauryl sulfate) depending on the storage condition of the pharmaceutical raw material for a formulation.
<11> A method for sorting a pharmaceutical raw material for a formulation consisting essentially of sodium lauryl sulfate, the method comprising:
   a) sorting a lot or packaging unit of the pharmaceutical raw material for a formulation which have been stored in a predetermined condition, and
   b) skipping detection of an impurity in the pharmaceutical raw material for a formulation which is sorted out in step a.
<12> The sorting method according to <11>, wherein the condition includes 30°C to 40°C for 47 days or less.
<13> The sorting method according to <11>, wherein the condition includes 30°C to 40°C for 47 days or less, and if the temperature exceeds 40°C, a cumulative sum of time at which the temperature is beyond 40°C to 60°C or less falls within 48 hours.
<13a> The sorting method according to any one of <11> to <13>, wherein the condition is 40°C.
<13b> The sorting method according to any one of <11> to <13a>, wherein the condition is 45 days.
<14> The sorting method according to any one of <11> to <13b>, wherein the condition is a storage condition during transportation.

Another embodiment of the present invention relates to a method for evaluating the quality of a pharmaceutical formulation by pretreating the pharmaceutical formulation under an accelerated condition.
<16> A method for evaluating the quality of a pharmaceutical formulation containing sodium lauryl sulfate, the method comprising detecting an impurity after pretreating the pharmaceutical formulation containing sodium lauryl sulfate under an accelerated condition.
<17> The quality evaluation method according to <16>, wherein the pharmaceutical formulation contains a compound represented by formula (I) 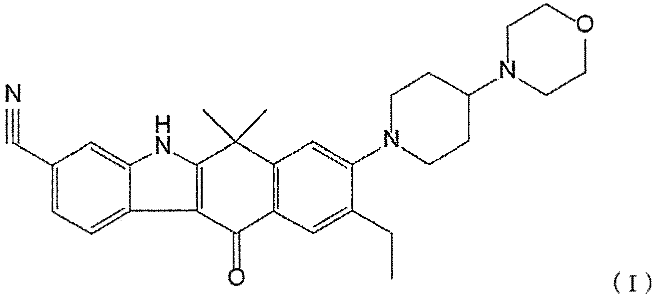 or a salt thereof.
<18> The quality evaluation method according to either one of <16> and <17>, wherein the accelerated condition is a temperature condition of 55 to 65°C.
<19> The quality evaluation method according to any one of <16> to <18>, wherein the accelerated condition is a temperature condition of about 60°C.
<20> The quality evaluation method according to any one of <16> to <19>, wherein the impurity is a sulfate ion, dodecanol or a sodium lauryl sulfate-dodecanol complex.
<21> The quality evaluation method according to any one of <16> to <20>, wherein the impurity is detected by X-ray powder diffraction, ion exchange chromatography, gas chromatography or quantitative NMR.
<22> The quality evaluation method according to any one of <16> to <21>, wherein the impurity is dodecanol, and the impurity is detected by gas chromatography.
<23> The quality evaluation method according to any one of <16> to <22>, wherein the impurity is a sulfate ion, and the impurity is detected by ion exchange chromatography.
<24> The quality evaluation method according to any one of <16> to <23>, wherein the pretreatment is performed under the accelerated condition for 2 days or more.
<25> The quality evaluation method according to any one of <16> to <24>, wherein the pretreatment is performed under the accelerated condition for 7 days.
<26> The quality evaluation method according to any one of <16> to <25>, wherein a pharmaceutical formulation in which 2% or less of dodecanol/sodium lauryl sulfate (mol/mol%) or a sulfate ion/sodium lauryl sulfate (mol/mol%) as the impurity is present, is sorted out.

Another embodiment of the present invention relates to a method for manufacturing a pharmaceutical formulation of alectinib, the method comprising a step of sorting sodium lauryl sulfate of a pharmaceutical raw material for a formulation by pretreating it under an accelerated condition.
<27> A method for manufacturing a pharmaceutical formulation containing sodium lauryl sulfate, the method comprising:
   a) pretreating sodium lauryl sulfate of a pharmaceutical raw material for a formulation under an accelerated condition;
   b) detecting an impurity after the pretreatment of step a to sort out a pharmaceutical raw material for a formulation in which the content of a sulfate ion as the impurity is 1 mg/L or less; and
   c) manufacturing a pharmaceutical formulation by using the pharmaceutical raw material for a formulation sorted out by step b.
<28> The production method according to <27>, wherein the pharmaceutical formulation comprises a compound represented by formula (I) 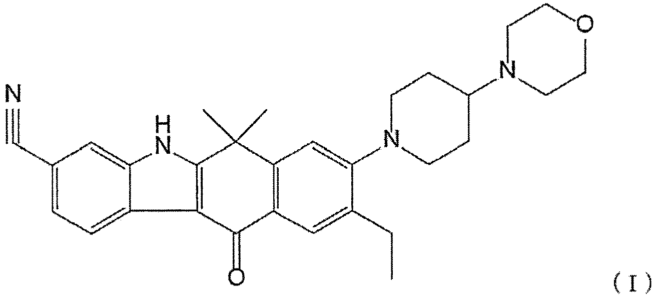 or a salt thereof.
<29> A method for manufacturing a pharmaceutical formulation containing a compound represented by formula (I) 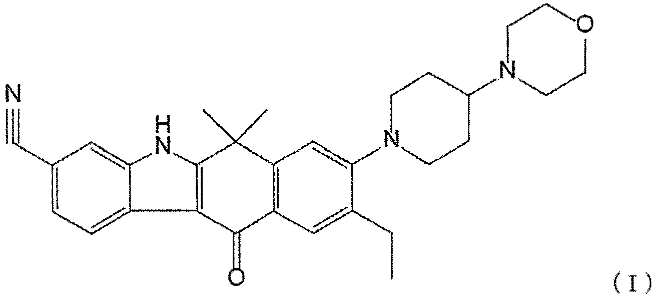 or a salt thereof and sodium lauryl sulfate, the method comprising:
   a) manufacturing the pharmaceutical formulation by using a pharmaceutical raw material for a formulation consisting essentially of sodium lauryl sulfate;
   b) pretreating the pharmaceutical formulation produced in step a; and
   c) detecting an impurity after the pretreatment of step b to sort out a pharmaceutical formulation in which the content of dodecanol/sodium lauryl sulfate (mol/mol%) or sulfate ion/sodium lauryl sulfate (mol/mol%) as the impurity is 2% or less.

Another embodiment of the present invention relates to a pharmaceutical raw material for a formulation or a pharmaceutical formulation thereof, alectinib-containing composition or a pharmaceutical formulation thereof containing an impurity measured by pretreating sodium lauryl sulfate of a pharmaceutical raw material for a formulation under an accelerated condition, in a predetermined ratio.
<30> A pharmaceutical composition containing a compound represented by formula (I) 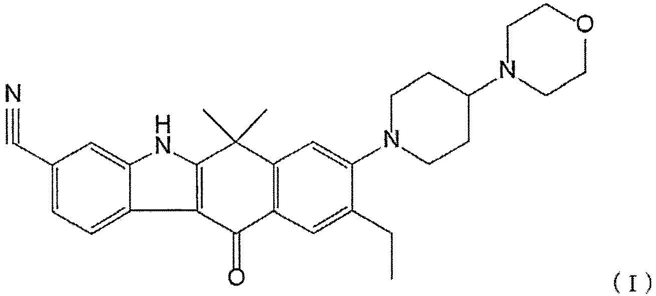 or a salt thereof and sodium lauryl sulfate, wherein dodecanol/sodium lauryl sulfate or sulfate ion/sodium lauryl sulfate is contained in a ratio such that it causes no effect on dissolution behavior of a pharmaceutical formulation containing the composition when the formulation is measured after a pretreatment under an accelerated condition.
<31> The composition according to <30>, wherein the ratio of dodecanol/sodium lauryl sulfate (mol/mol%) or sulfate ion/sodium lauryl sulfate (mol/mol%) in the composition, as measured by X-ray powder diffraction, ion exchange chromatography, gas chromatography or quantitative NMR is 2% or less.
<32> A pharmaceutical formulation containing a compound represented by formula (I) 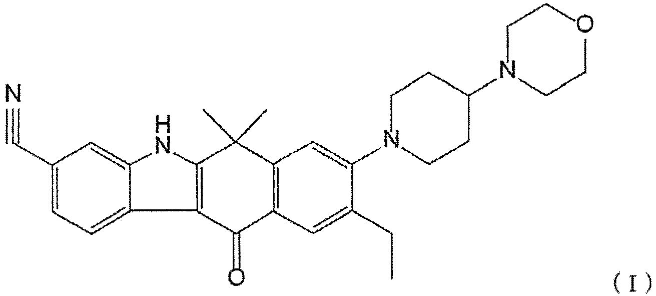 or a salt thereof and sodium lauryl sulfate, wherein dodecanol/sodium lauryl sulfate or sulfate ion/sodium lauryl sulfate is contained in a ratio such that it causes no effect on dissolution behavior of the formulation when the composition is measured after a pretreatment under an accelerated condition.
<33> The formulation according to <32>, wherein the ratio of dodecanol/sodium lauryl sulfate (mol/mol%) or sulfate ion/sodium lauryl sulfate (mol/mol%) in the formulation is 2% or less, as measured by X-ray powder diffraction, ion exchange chromatography, gas chromatography or quantitative NMR.
<34> A pharmaceutical formulation the method comprising sodium lauryl sulfate, wherein the formulation contains dodecanol/sodium lauryl sulfate or sulfate ion/sodium lauryl sulfate in the ratio such that it causes no effect on dissolution behavior when the formulation is measured after a pretreatment under an accelerated condition.
<35> A pharmaceutical raw material for a formulation consisting essentially of sodium lauryl sulfate, wherein the content of the sulfate ion in the material is 1 mg/L or less when the pharmaceutical raw material for a formulation is pretreated under an accelerated condition and thereafter measured by ion exchange chromatography.

The following embodiments are also included in the present invention.
(1) A method of sorting a pharmaceutical raw material for a formulation consisting essentially of sodium lauryl sulfate, the method comprising detecting an impurity after pretreating the pharmaceutical raw material for a formulation under an accelerated condition.
(2) The sorting method according to (1), wherein the accelerated condition is a temperature condition of 65 to 75°C.
(3) The sorting method according to (1) or (2), wherein the accelerated condition is a humidity condition of less than 79%RH.
(4) The sorting method according to any one of (1) to (3), wherein the accelerated condition is a humidity condition of about 10%RH.
(5) The sorting method according to any one of (1) to (4), wherein the accelerated condition includes a temperature condition of about 70°C and a humidity condition of about 10%RH or less.
(6) The sorting method according to any one of (1) to (3), wherein the impurity is a sulfate ion, dodecanol or a sodium lauryl sulfate-dodecanol complex.
(7) The sorting method according to any one of (1) to (6), wherein the impurity is detected by X-ray powder diffraction, ion exchange chromatography, gas chromatography or quantitative NMR.
(8) The sorting method according to any one of (1) to (7), wherein the impurity is a sulfate ion, and the impurity is detected by ion exchange chromatography.
(9) The sorting method according to any one of (1) to (8), wherein the pretreatment is performed under the accelerated condition for 2 days or more.
(10) The sorting method according to any one of (1) to (8), wherein the pretreatment is performed under the accelerated condition for 4 days.
(11) A method for evaluating the quality of a pharmaceutical formulation, the method comprising detecting an impurity after pretreating the pharmaceutical formulation under an accelerated condition.
   (11a) The quality evaluation method according to (11), wherein the pharmaceutical formulation contains sodium lauryl sulfate.
(12) The quality evaluation method according to (11) or (11a), wherein the accelerated condition is a temperature condition of about 60°C.
(13) The quality evaluation method according to (11), (11a) or (12), wherein the pharmaceutical formulation contains a compound represented by formula (I) 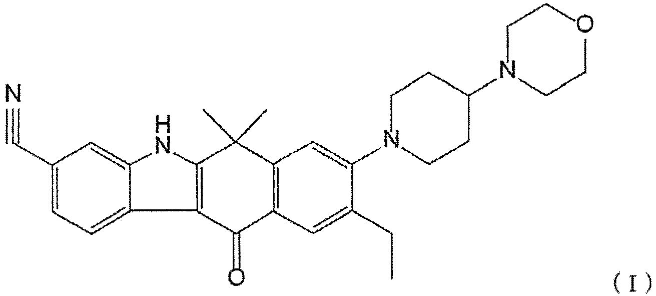 or a salt thereof.
(14) A method for manufacturing a pharmaceutical formulation containing sodium lauryl sulfate, the method comprising:
   a) pretreating sodium lauryl sulfate of a pharmaceutical raw material for a formulation under an accelerated condition;
   b) detecting an impurity after the pretreatment of step a to sort out a pharmaceutical raw material for a formulation in which the content of a sulfate ion as the impurity is 1 mg/L or less; and
   c) manufacturing a pharmaceutical formulation by using the pharmaceutical raw material for a formulation sorted out by step b.
(15) The production method according to (14), wherein the pharmaceutical formulation contains a compound represented by formula (I) 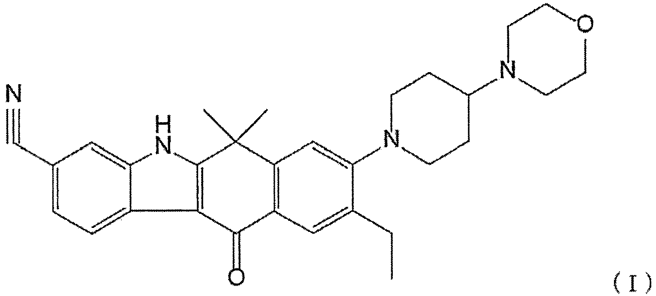 or a salt thereof.

### Advantageous Effects of Invention

According to the method of the present invention, it is possible to determine or sort out the quality of sodium lauryl sulfate (SLS) of a pharmaceutical raw material for a formulation, and the pharmaceutical composition produced by using the pharmaceutical raw material for a formulation sorted out by the method of the present invention has excellent stability and can provide a pharmaceutical formulation having high quality.

According to the sorting method of the present invention, it is possible to sort out a pharmaceutical raw material for a formulation, from which a pharmaceutical formulation excellent in stability can be produced, by pretreating sodium lauryl sulfate (SLS) of a pharmaceutical raw material for a formulation under a predetermined accelerated condition. Alternatively, the sorting method of the present invention can skip impurity detection depending on the storage condition of the pharmaceutical raw material for a formulation.

According to the quality evaluation method of the present invention, the quality of the pharmaceutical formulation can be evaluated by detecting the amount of an impurity after pretreating sodium lauryl sulfate of a pharmaceutical raw material for a formulation under a predetermined accelerated condition. Alternatively, according to the quality evaluation method of the present invention, a pharmaceutical formulation containing sodium lauryl sulfate can be evaluated as having a quality, such that it is sufficient to skip impurity measurement, based on whether the formulation is manufactured or not by using sodium lauryl sulfate of a pharmaceutical raw material for a formulation which was sorted by the amount of impurity after pretreated under a predetermined accelerated condition.

According to the production method of the present invention, sodium lauryl sulfate of a pharmaceutical raw material for a formulation is pretreated under a predetermined accelerated condition, and the raw material having a low content of impurity is sorted out and subjected to manufacture of a pharmaceutical formulation. In this manner, a pharmaceutical formulation containing sodium lauryl sulfate having excellent stability can be produced.

According to the present invention, it is possible to provide a pharmaceutical raw material for a formulation useful for manufacturing a pharmaceutical formulation having excellent stability, or provide an alectinib-containing composition or a pharmaceutical formulation containing an impurity at a predetermined ratio or less so as not to affect dissolution behavior.

### Brief Description of Drawings

[Figure 1A] The figure is a graph showing the measurement results of X-ray powder diffraction of sodium lauryl sulfate (Lot 1) stored in temperature condition 1A.
[Figure 1B] The figure is a graph showing the measurement results of X-ray powder diffraction of sodium lauryl sulfate (Lot 2) stored in temperature condition 1A.
[Figure 1C] The figure is a graph showing the measurement results of X-ray powder diffraction of sodium lauryl sulfate (Lot 1) stored in temperature condition 1B.
[Figure 1D] The figure is a graph showing the measurement results of X-ray powder diffraction of sodium lauryl sulfate (Lot 2) stored in temperature condition 1B.
[Figure 2A] The figure is a graph showing the measurement results of X-ray powder diffraction of sodium lauryl sulfate (Lot 1) stored in humidity condition 2A.
[Figure 2B] The figure is a graph showing the measurement results of X-ray powder diffraction of sodium lauryl sulfate (Lot 2) stored in humidity condition 2A.
[Figure 2C] The figure is a graph showing the measurement results of X-ray powder diffraction of sodium lauryl sulfate (Lot 1) stored in humidity condition 2B.
[Figure 2D] The figure is a graph showing the measurement results of X-ray powder diffraction of sodium lauryl sulfate (Lot 2) stored in humidity condition 2B
[Figure 3] The figure is a graph showing the measurement results of X-ray powder diffraction of a sodium lauryl sulfate-dodecanol complex.
[Figure 4] The figure shows scanning electron micrographs of sodium lauryl sulfate and a sodium lauryl sulfate-dodecanol complex.
[Figure 5] The figure shows charts of a proton nuclear magnetic resonance spectrum of sodium lauryl sulfate and a sodium lauryl sulfate-dodecanol complex.
[Figure 6A] The figure shows the measurement results of X-ray powder diffraction of sodium lauryl sulfate (Lot 1) stored at a temperature of 70°C and a relative humidity of about 10% (a saturated aqueous solution of LiCI).
[Figure 6B] The figure shows the measurement results of X-ray powder diffraction of sodium lauryl sulfate (Lot 2) stored at a temperature of 70°C and a relative humidity of about 10% (a saturated aqueous solution of LiCI).
[Figure 7A] The figure shows the measurement results of X-ray powder diffraction of a formulation (Lot A) stored at a temperature condition 5A.
[Figure 7B] The figure shows the measurement results of X-ray powder diffraction of a formulation (Lot B) stored at a temperature condition 5A.
[Figure 7C] The figure shows the measurement results of X-ray powder diffraction of a formulation (Lot A) stored at a temperature condition 5B.
[Figure 7D] The figure shows the measurement results of X-ray powder diffraction of a formulation (Lot B) stored at a temperature condition 5B.
[Figure 7E] The figure shows the measurement results of X-ray powder diffraction of a formulation (Lot A) stored at a temperature condition 5C.
[Figure 7F] The figure shows the measurement results of X-ray powder diffraction of a formulation (Lot B) stored at a temperature condition 5C.
[Figure 8A] The figure shows dissolution test profile of a formulation (Lot C) stored at a temperature of 60°C.
[Figure 8B] The figure shows dissolution test profile of a formulation (Lot D) stored at a temperature of 60°C.
[Figure 9A] The figure shows the measurement results of X-ray powder diffraction of a formulation (Lot C) stored at a temperature of 60°C.
[Figure 9B] The figure shows the measurement results of X-ray powder diffraction of a formulation (Lot D) stored at a temperature of 60°C.
[Figure 10A] The figure shows dissolution test profile of a formulation (Lot E) stored at a temperature of 60°C.
[Figure 10B] The figure shows dissolution test profile of a formulation (Lot F) stored at a temperature of 60°C.
[Figure 10C] The figure shows dissolution test profile of a formulation (Lot G) stored at a temperature of 60°C.
[Figure 10D] The figure shows dissolution test profile of a formulation (Lot H) stored at a temperature of 60°C.
[Figure 10E] The figure shows dissolution test profile when a formulation (Lot I) was stored at a temperature of 60°C.
[Figure 10F] The figure shows dissolution test profile of a formulation (Lot J) stored at a temperature of 60°C.
[Figure 10G] The figure shows dissolution test profile of a formulation (Lot K) stored at a temperature of 60°C.
[Figure 11] The figure is a graph showing the measurement results of dodecanol by GC/MS when the formulations (Lot A and Lot B) were stored at a temperature of 60°C.

### Description of Embodiments

Now, the present invention will be described below.

In the present invention, "sodium lauryl sulfate" is a substance known as an additive of pharmaceutical formulations; and particularly used as a dissolution aid for a poorly soluble drug, alectinib, for constituting a (pharmaceutical) composition of a pharmaceutical formulation of the invention. Sodium lauryl sulfate is known to exist in the form of a monohydrate, a 1/2 hydrate, a 1/8 hydrate, and a non-solvate (literature: Journal of Crystal Growth 263 (2004) 480-490). Sodium lauryl sulfate is usually stored at room temperature and preferably used within a year from production. Any one of the forms can be used as the pharmaceutical raw material for a formulation of the present invention.

The "pharmaceutical raw material for a formulation" refers to a substantially pure single compound (for example, sodium lauryl sulfate) to be used as an additive for a pharmaceutical formulation containing an active ingredient such as alectinib. The pharmaceutical raw material for a formulation refers to a raw material for use in industrial manufacturing of the pharmaceutical formulation and containing a desired compound in an amount in the order of kilograms or more. The term "substantially pure" refers to containing a desired compound to the extent that it is generally available for distribution although an impurity is allowed to exist to some extent. In the present invention, the pharmaceutical raw material for a formulation may be a commercially available material purchased from a manufacturer or produced by oneself or by an entrusting company. The pharmaceutical raw material for a formulation may be used immediately after purchase or production or at an interval of a predetermined period, for example, one year or more, from purchase/production.

The "pharmaceutical raw material for a formulation consisting essentially of sodium lauryl sulfate" refer to substantially pure sodium lauryl sulfate, which means that a small amount of its impurities, such as a sulfate ion, dodecanol or a sodium lauryl sulfate-dodecanol complex can be existed.

The term "sorting" refers to selecting a pharmaceutical raw material for a formulation or a pharmaceutical formulation having a higher purity; more specifically, refers to selecting a lot or a packaging unit of a pharmaceutical raw material for a formulation and a pharmaceutical formulation having a lower impurity-content through measurement of the impurity contents in individual lots or package units thereof. Sorting a pharmaceutical raw material for a formulation refers to preferably selecting a pharmaceutical raw material for a formulation having a higher purity; further preferably selecting a lot or packaging unit of a pharmaceutical raw material for a formulation having an impurity content adapted to the purity test described in the Japanese Pharmacopoeia (17th revision). Note that, the "lot" refers to a minimum production/shipping unit of a raw material or an article produced under the same condition and in a predetermined period. The "packaging unit" refers to a pharmaceutical raw material for a formulation or a pharmaceutical formulation (one lot or more) packaged in the unit of a predetermined weight or number. More preferably, when the pharmaceutical raw material for a formulation is sodium lauryl sulfate and the impurity is a sulfate ion, sorting is made by selecting the lot or packaging unit containing a sulfate ion in an amount of 5 mg/L or less, preferably 3 mg/L or less, and most preferably 1 mg/L or less in a solution (obtained by dissolving a sample in water) when measured by ion exchange chromatography; or by selecting the lot or packaging unit, in which the content of a sulfate ion of 1 mg/L or less in a solution of about 100 mg/L of a sample in water.

Sorting of a pharmaceutical formulation containing dodecanol or a sodium lauryl sulfate-dodecanol complex as an impurity is made by selecting a lot or packaging unit having a content of dodecanol/sodium lauryl sulfate (mol/mol%) of less than 5%, preferably less than 3% and most preferably less than 2%, as measured by X-ray powder diffraction, ion exchange chromatography, gas chromatography or quantitative NMR.

Ion chromatography is a kind of liquid chromatography mainly used in measuring ion species components. Using an eluent as a mobile phase and, e.g., an ion exchanger as a stationary phase to be charged in a column, ion species (components) in a sample solution can be separated and quantified. In gas chromatography, separation is made based on the interaction between a stationary phase and a substance. Qualitative determination can be made based on the time required for a substance to reach a detector; whereas, quantitative determination can be made based on the amount of signal from the detector. Measurement by X-ray powder diffraction can be made, for example, in accordance with a common method such as "X-ray powder diffraction method" described in the Japanese Pharmacopoeia (17th revision).

Quantitative NMR is a quantification method carried out based on the principle that the area of a signal peak observed in an NMR spectrum is proportional to the number of hydrogen atoms contained in a sample and does not depend on the chemical structure of a substance. Accordingly, an absolute amount of a component to be analyzed can be determined by using a substance (except the component to be analyzed) having a purity already known, as a standard substance. Preferably, gas chromatography or ion chromatography using an anion exchange column is used. A mobile phase, column and detector of ion chromatography are preferably as follows:

Mobile phase (eluate): 5 mM Na₂CO₃ solution or 20 mM NaOH solution
Column: Metrosep A Supp 7-250/4.0 (manufactured by Metrohm) can be used if a 5 mM Na₂CO₃ solution is used, or Ion Pac AS11-HC (diameter: 4 mm, lengths: 250 mm (manufactured by Thermo Scientific) if 20 mM NaOH solution is used
Detector: Conductance meter.

Preferable column and measurement condition of gas chromatography are as follows:
Column: HP-5MS UI, 0.25 mm ID × 30 m, 0.25 µm (manufactured by Agilent)
Injection volume: 1.0 µL
Split ratio: 1: 10
Column temperature: 40°C (1 min) -> (20°C/min) -> 180°C -> (5°C/min) -> 190°C -> (50°C/min) -> 300°C (4 min)
Injector temperature: 300°C
Interface temperature: 30°C
Carrier gas: He
Flow rate: 1 mL/min
Ion voltage: 70 eV
Ionization mode: ESI-
Measurement mode: SIM
Monitor ion (m/z): 83.0

The "accelerated condition" refers to a condition severer than the condition for ordinary storage; more specifically, conditions where temperature, humidity and etc., are severer than those in ordinary storage. More specifically, the temperature employed as the accelerated condition for a pharmaceutical raw material for a formulation is 50°C or more, preferably about 60°C or more, more preferably, about 60°C to 80°C, further preferably about 65°C to 75°C and most preferably about 70°C. The humidity condition employed as the accelerated condition is 80%RH or less, preferably less than 79%RH and more preferably less than about 10%RH.

As long as a pharmaceutical raw material for a formulation is stored in a predetermined condition, it is possible to suppress the ratio of the impurity to be generated. Because of this, if a pharmaceutical formulation containing a pharmaceutical raw material for a formulation stored in the predetermined condition is selected, measurement for impurity can be skipped.

The accelerated temperature condition of a pharmaceutical formulation is 50°C or more, preferably about 50°C to 70°C or more, more preferably, about 55°C to 65°C and most preferably about 60°C.

Note that, "storing" refers to storage (safekeeping) in, e.g., a warehouse, a storehouse or a cargo where temperature and humidity can be controlled for a predetermined period from production of a pharmaceutical raw material for a formulation to use thereof. Transportation requiring one day or more is also included.

In the present invention, the "predetermined condition" refers to the temperature and the number of days during a storage period. More specifically, the temperature is 40°C or less and the number of days is 47 days or less, preferably 30°C to 40°C and 47 days or less, more preferably 30°C to 40°C and 45 days.

The phrase "exceeding a predetermined condition" as to temperature refers to a storage temperature of higher than 40°C during storage. Note that even if the temperature exceeds the predetermined temperature condition, as long as a predetermined range is satisfied, the temperature in excess of the condition has less effect on impurity generation. In this case, impurity measurement can be skipped. To describe it more specifically, even if the temperature exceeds 40°C, if a cumulative sum obtained by adding time periods during which the temperature is beyond 40°C to 60°C or less, falls within 48 hours, impurity measurement can be skipped.

The phrase "exceeding a predetermined condition" as to the number of days refers to 47 days and preferably beyond 45 days at a temperature of 40°C or less. Note that, if storage is made at room temperature to 30°C in a period of one year or less, similarly, impurity measurement can be skipped.

As described above, the case where storage is made beyond the condition (additional condition) allowing impurity measurement to skip corresponds to the case where a "predetermined condition is exceeded".

Note that, in the specification of the present application, the term "about" means acceptable range of ± 10%.

The term "pretreatment" refers to a predetermined treatment applied to a pharmaceutical raw material for a formulation before it is used for manufacturing a pharmaceutical formulation. More specifically, the predetermined treatment refers to allowing a raw material to stand still under the accelerated condition for a predetermined period. The term "predetermined period" for a pharmaceutical raw material for a formulation refers to one day or more, preferably 2 days or more, more preferably, 3 days or more and further preferably 4 days. The term "predetermined period" for a pharmaceutical formulation refers to 5 days or more, preferably 7 days or more, further preferably 7 days or more 14 within days and most preferably 7 days or less.

The "impurity" refers to a substance except a pharmaceutical raw material for a formulation contained in individual lots or packaging units of the pharmaceutical raw material for a formulation, for example a raw material for manufacturing a pharmaceutical raw material for a formulation, a decomposed substance or complex thereof; or a raw material for manufacturing a pharmaceutical raw material for a formulation contained in a pharmaceutical formulation, a decomposed substance or a complex thereof. For example, if the pharmaceutical raw material for a formulation is sodium lauryl sulfate or if a pharmaceutical formulation contains sodium lauryl sulfate, the impurity is mainly a sulfate ion, dodecanol or a sodium lauryl sulfate-dodecanol complex. A sulfate ion as an impurity of a pharmaceutical raw material for a formulation can be measured by ion exchange chromatography, and the acceptable concentration of a sulfate ion is 5 mg/L or less, preferably 3 mg/L or less, most preferably 1 mg/L or less in a solution obtained by dissolving a sample in water. Dodecanol as an impurity of a pharmaceutical formulation can be measured by the same method as employed in "sorting", such as X-ray powder diffraction, ion exchange chromatography, gas chromatography or quantitative NMR. The ratio of dodecanol/sodium lauryl sulfate (mol/mol%) is preferably 2% or less.

The "ratio such that it causes no effect on dissolution behavior" refers to the ratio of impurity when the difference in dissolution between a pharmaceutical formulation containing a predetermined amount of impurity and a pharmaceutical formulation containing no impurity falls within 10%. For example, when pharmaceutical formulations different in impurity content are subjected to the dissolution test (which is carried out by using a dissolution test solution, Solution 1 (900 mL) containing 7% of polyoxyethylene (10) octyl phenyl ether and defined in the 16th revised the Japanese Pharmacopoeia, in accordance with paddle method defined in the dissolution test of the 16th revised Japanese Pharmacopoeia, at a rotation rate of 100 per minute and at 37°C), if the drug dissolution rate is compared to that of a pharmaceutical formulation containing no impurity at a predetermined time point, (preferably a time point of 75 minutes), the impurity amount of a formulation whose difference in drug dissolution rate falls within the range of 10%, can be obtained. More specifically, the impurity (dodecanol/sodium lauryl sulfate (mol/mol%)) in a pharmaceutical formulation containing sodium lauryl sulfate, is preferably less than 2%.

The "detection" refers to qualitatively or quantitatively determining a substance (impurity) except the raw material contained in individual lots or packaging units of a pharmaceutical formulation or a pharmaceutical raw material for a formulation. For example, if the pharmaceutical raw material for a formulation is sodium lauryl sulfate, or if a pharmaceutical formulation contains sodium lauryl sulfate, the "detection" refers to qualitatively or quantitatively determining the presence or absence of an impurity such as sulfate ion, dodecanol or a sodium lauryl sulfate-dodecanol complex by, e.g., X-ray powder diffraction, ion exchange chromatography, gas chromatography or quantitative NMR, in the same manner as in "sorting".

The "pharmaceutical formulation" refers to a dosage form suitable for intake formed with a pharmaceutical composition containing a biologically active substance as an active ingredient and a pharmaceutical acceptable additive(s). In the present invention, an oral formulation is preferable. The "oral formulation" refers to a formulation that can be orally administered and an active ingredient of the formulation is mainly absorbed through the intestinal tract. Examples of the oral formulation include a solid formulation and a liquid formulation. In the present invention, a solid formulation is preferable. More specifically, examples of the solid formulation include tablets, capsules, powders, lozenges and chewable agents. Of them, tablets are preferable. A pharmaceutical formulation of the present invention may contain additives such as an excipient, a lubricant, a coating agent, a binder, a disintegrant, a stabilizer, a flavoring agent and a diluent usually used in pharmaceutical formulations. A pharmaceutical formulation of the present invention preferably contains, a compound represented by formula (I) or a salt thereof, and more preferably, alectinib hydrochloride.

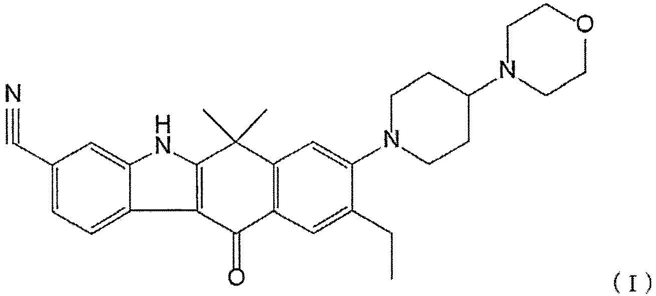

In the present invention, a pharmaceutical formulation to be used for quality evaluation may be produced by oneself or may be a commercially available formulation.

The "pharmaceutical composition" refers to a mixture of at least two types of substances including a biologically active substance for use in, e.g., therapy/prevention of a disease as an active ingredient and a pharmaceutically acceptable additive(s). As the biologically active substance in the present invention is preferably a compound represented by formula (I) or a salt thereof.

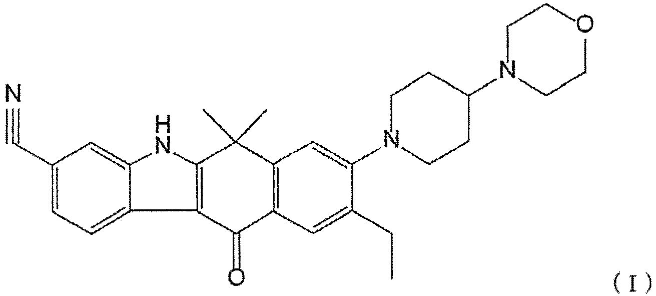

As the additive, preferably sodium lauryl sulfate is contained and, further, lactose hydrate, hydroxypropyl cellulose, carmellose calcium and magnesium stearate may be contained.

The "quality evaluation method" refers to a method of evaluating the quality of a pharmaceutical formulation by qualitatively or quantitatively measuring impurity in the pharmaceutical formulation. Qualitative or quantitative impurity measurement can be made by, e.g., X-ray powder diffraction, ion exchange chromatography, gas chromatography or quantitative NMR. Preferably, in the quality evaluation method, the quality of the pharmaceutical formulation is evaluated by the content of an impurity in a pharmaceutical formulation. More specifically, a pharmaceutical formulation having a content ratio of dodecanol/sodium lauryl sulfate (mol/mol%) of 5% or less, preferably 3% or less, and more preferably 2% or less is evaluated as a pharmaceutical formulation having preferable quality, wherein the content ratio of impurity in a pharmaceutical formulation is obtained by using a method employed in "sorting".

The "compound represented by formula (I)" refers to a compound represented by formula (I) 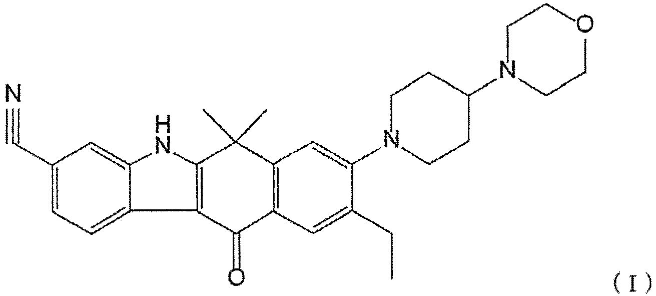 that is 9-ethyl-6,6-dimethyl-8-(4-morpholin-4-yi-piperidin-1-yl)-11-oxo-6,11-dihydro-5H-benzo[b]carbazole-3-carbonitrile, generic name alectinib.

A "salt" of a compound represented by formula (I) is preferably a pharmaceutical acceptable salt. Examples of "the pharmaceutical acceptable salt" include a hydrochloride, a hydrobromide, a hydroiodide, a phosphate, a phosphonate, a sulfate, a sulfonate such as methanesulfonate and p-toluene sulfonate; a carboxylate such as an acetate, a citrate, a malate, a tartrate, a succinate and a salicylate; an alkali metal salt such as a sodium salt, a potassium salt; an alkaline earth metal salt such as a magnesium salt, a calcium salt; and an ammonium salt such as an ammonium salt (NH₄X: X is a monovalent acid group), an alkylammonium salt, a dialkylammonium salt, a trialkylammonium salt and a tetraalkylammonium salt.

A hydrochloride is preferable and a monohydrochloride is the most preferable.

A compound represented by formula (I) or a salt thereof can be produced by a method commonly known in the art (for example, method described in Patent Literature 2).

A monohydrochloride of a compound represented by formula (I) may be amorphous or crystalline state. In the case of a crystal, a crystal (type I crystal) having peaks at diffraction angles (2θ) of around 8.4°, 14.0°, 16.7°, 18.8° and 23.3° in a X-ray powder diffraction pattern. A monohydrochloride of a compound represented by formula (I) may be a hydrate. An amorphous monohydrochloride of a compound represented by formula (I) can be produced by a method described in WO2016/021707 and a crystal thereof having the peaks can be produced by a method described in WO2015/163447.

The content of a compound represented by formula (I) or a salt thereof is 20 to 70 wt%, preferably 35 to 60 wt% and further preferably 45 to 50 wt% based on the total amount of the composition in terms of free body.

A formulation obtained by the manufacturing method of the present invention contains a compound represented by formula (I) or a salt thereof in an amount of 150 mg to 800 mg, preferably 150 mg to 400 mg, and particularly preferably 150 mg to 300 mg per unit formulation, in terms of free body. More specifically, a 150 mg capsule and 150 mg, 300 mg and 600 mg tablets are mentioned.

### Examples

### Example 1: Setting of pre-treatment condition (temperature) of sodium lauryl sulfate evaluation method

Samples (appropriate amount) of different lots, Lot 1 (production date: 2015.12.22) and Lot 2 (production date: 2017.3.10)) of sodium lauryl sulfate, which were obtained from a single manufacturer and produced on different days, were placed in glass vials and stored in an airtight system under condition 1A (temperature 60°C) and condition 1B (temperature 70°C). Samples before storage, stored for 3 days and 4 day were subjected to X-ray powder diffraction (XRPD). Measurement was performed in the conditions shown below.

X-ray powder diffraction method:
Measuring device: X'Pert-Pro MPD (manufactured by PANalytical)
Anti-cathode: Cu
Tube voltage: 45 kV
Tube current: 40 mA
Step width: 0.02
Scan axis: 2θ
Sampling time per step: 43 seconds
Scan range: 3 to 40°

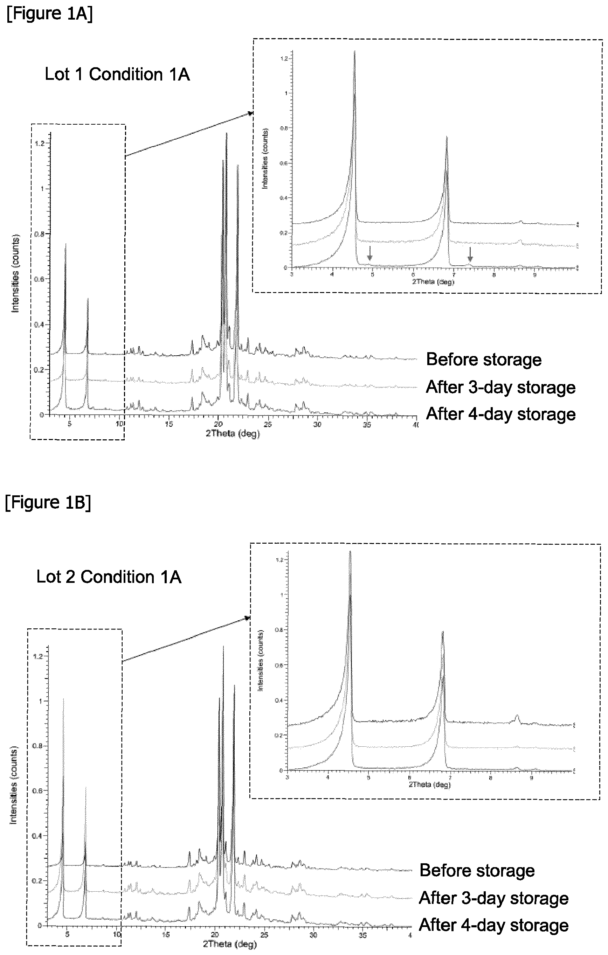

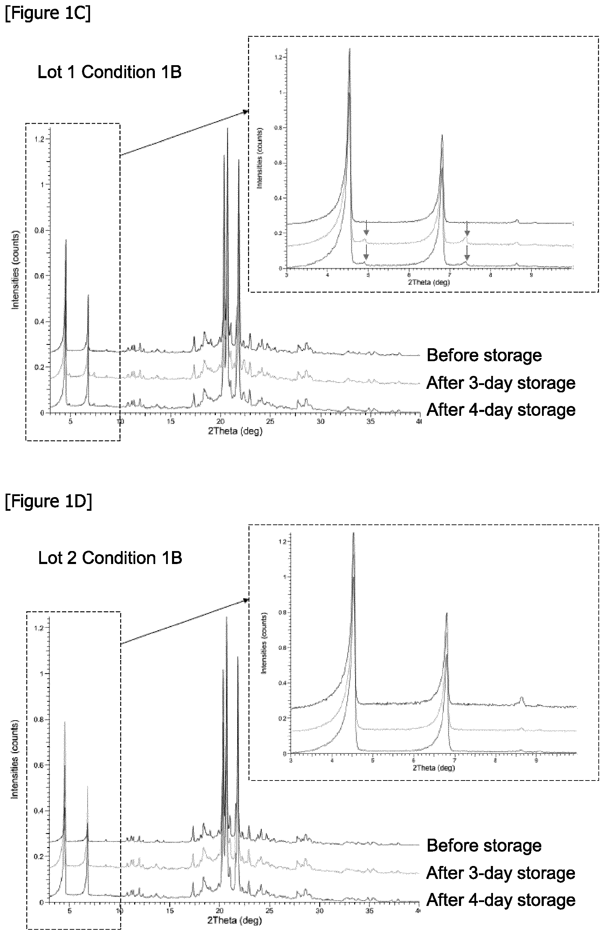

X-ray powder diffraction patterns of Lot 1 and Lot 2 samples stored in condition 1A and condition 1B are shown in Figure 1. XRPD patterns of Lot 2 samples stored in both condition 1A and condition 1B did not change until after 4-day storage.

In contrast, in the XRPD pattern of a Lot 1 sample in condition 1A after 4-day storage and the pattern of a Lot 1 samples in condition 1B after 3-day storage, new peaks were observed at diffraction angles (2θ) of around 4.9° and 7.4°.

The storage periods during which new peaks were first detected in Lot 1 and Lot 2 XRPD patterns in condition 1A and condition 1B are shown below.

### Condition 1A

Lot 1: after 4-day storage
Lot 2: not detected after 4-day storage

### Condition 1B

Lot 1: after 3-day storage
Lot 2: not detected after 4-day storage

From the results, it was determined that condition 1B (high in temperature) is the condition where the sodium lauryl sulfate Lot 1 and Lot 2 can be more quickly evaluated. Then, study on humidity condition (Example 2) was started.

### Example 2: Setting of pre-treatment condition (humidity) of sodium lauryl sulfate evaluation method

Samples (about 250 mg) of different lots, Lot 1 and Lot 2, of sodium lauryl sulfate used in Example 1 were placed in glass vials and stored in an open system under condition 2A (temperature 70°C, relative humidity about 79% (A saturated aqueous solution of NaCl)) and condition 2B (temperature 70°C, relative humidity about 10% (a saturated aqueous solution of LiCI). Samples before storage, stored for a single day, 2 days and 3 days^{∗1} in condition 2A and samples before storage, for a single day, 2 days, 3 days and 6 days in condition 2B were subjected to X-ray powder diffraction. Measurement was performed in the conditions shown in Example 1.

^{∗}1: Since Lot 1 samples stored in condition 2A for 2-days storage and 3-days storage changed to a liquid state, these samples were not subjected to measurement by X-ray powder diffraction.

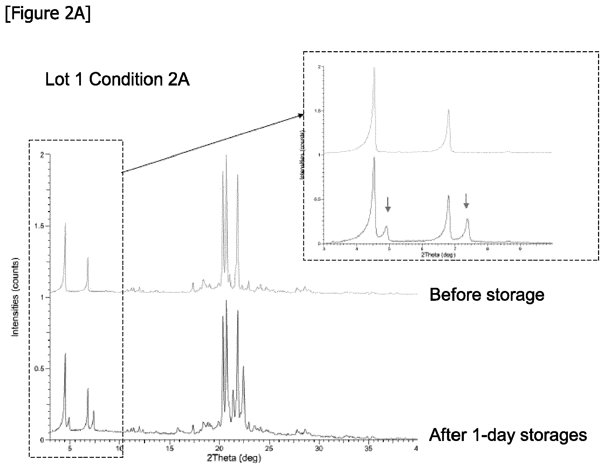

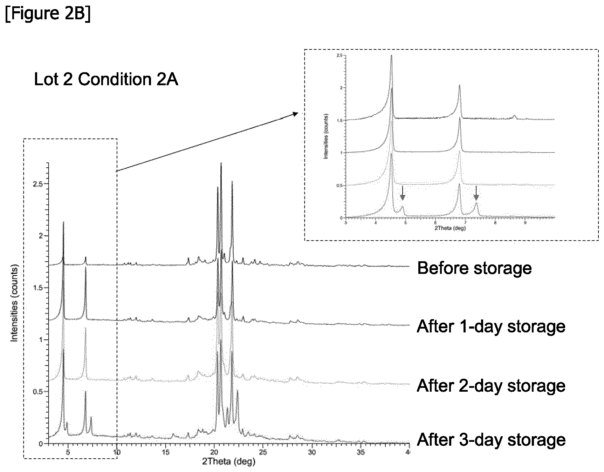

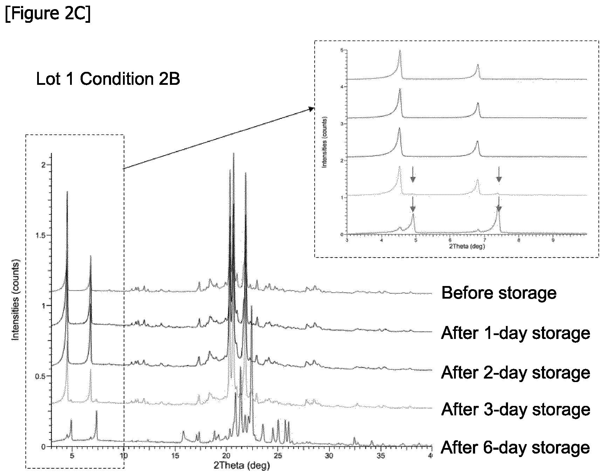

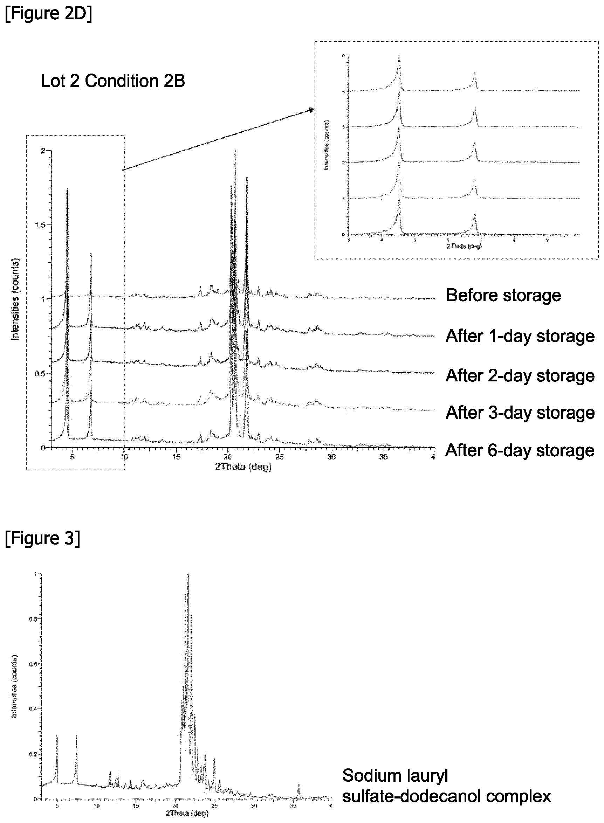

X-ray powder diffraction patterns of Lot 1 and Lot 2 samples stored in condition 2A and condition 2B are shown in Figure 2. XRPD patterns of Lot 2 samples stored in condition 2B did not change after 6-day storage; whereas, in the pattern of Lot 1 sample after 3-day storage, new peaks were observed at diffraction angles (2θ) of about 4.9° and 7.4°. In contrast, in condition 2A, in the patterns of Lot 1 samples after 1-day storage and the patterns of Lot 2 samples after 3-days storage, peaks were observed.

The storage periods during which new peaks were first detected in Lot 1 and Lot 2 XRPD patterns in condition 2A and condition 2B are shown below.

### Condition 2A

Lot 1: after 1-day storage
Lot 2: after 3-day storage

### Condition 2B

Lot 1: after 3-day storage
Lot 2: not detected before 6-day storage

From the results, condition 2B (relative humidity is low) was determined as the condition where the sodium lauryl sulfate Lot 1 and Lot 2 can be distinguished and used for sorting sodium lauryl sulfate samples.

### Example 3: Identification of impurity during storage period of sodium lauryl sulfate

Sodium lauryl sulfate (about 85 mg) was added in dodecanol (about 2 mL). The mixture was stirred for about 12 hours while keeping the temperature at 30°C. The solid precipitate was filtered to obtain a white crystal. The crystal (a sodium lauryl sulfate-dodecanol complex) was subjected to X-ray powder diffraction performed in the conditions of Example 1. The measurement results are shown in Figure 3. The peaks observed at diffraction angles (2θ) of around 4.9° and 7.4° in the pattern of a sodium lauryl sulfate-dodecanol complex accurately coincided with the peaks of unknown substances observed in storage samples of sodium lauryl sulfate in Example 1 and Example 2.

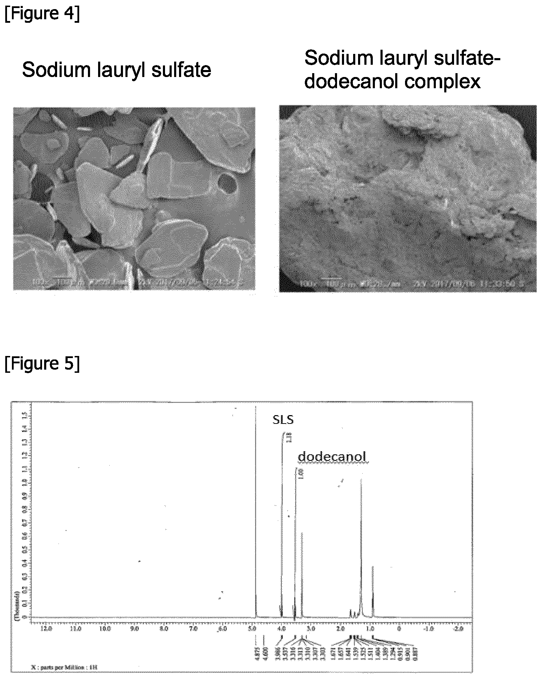

Samples of sodium lauryl sulfate and a sodium lauryl sulfate-dodecanol complex were observed by a scanning electron microscope in the following conditions. The results are shown in Figure 4. Sodium lauryl sulfate looks a thin plate-like crystal; whereas, a sodium lauryl sulfate-dodecanol complex differs in shape.

Measurement conditions by scanning electron microscope:
Ion sputtering device: JFC-1500 (manufactured by JEOL)
Measuring device: SEM VE-9800 (manufactured by Keyence)
Acceleration voltage: 2 kV
Magnification of objective lens: 100X

To check the molar ratio of sodium lauryl sulfate and dodecanol in a sodium lauryl sulfate-dodecanol complex, the sodium lauryl sulfate-dodecanol complex (about 6 mg) was dissolved in 1 mL of methanol-d4 (99.8%) and subjected to measurement of a proton nuclear magnetic resonance spectrum (1H-NMR spectrum) performed in the conditions shown below.
Measuring device: ECP500 (manufactured by JEOL)
Solvent: methanol-d4
X axis resolution: 0.22673 [Hz]
Number of scanning lines: 16
Relaxation delay 60s:

The proton nuclear magnetic resonance spectrum (chart) is shown in Figure 5. As a result of quantitative analysis, the molar ratio of sodium lauryl sulfate and dodecanol in the sodium lauryl sulfate-dodecanol complex was 1.18: 1.00.

From the results, it was found that sodium lauryl sulfate is decomposed during storage and forms a complex with one of degradation products, i.e., dodecanol.

### Example 4: Study of storage period in accelerated condition (XRPD, ion chromatography)

A sample of sodium lauryl sulfate (about 250 mg) was placed in a glass vial and stored in an open system at a temperature of 70°C and a relative humidity of about 10% (a saturated aqueous solution of LiCI). Samples of before storage, stored for a single day, 2 days, 3 days, 4 days, 7 days and 11 days were subjected to X-ray powder diffraction performed in the conditions of Example 1.

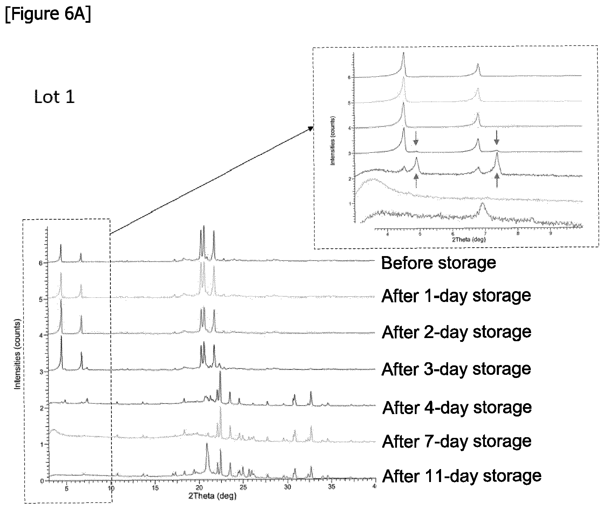

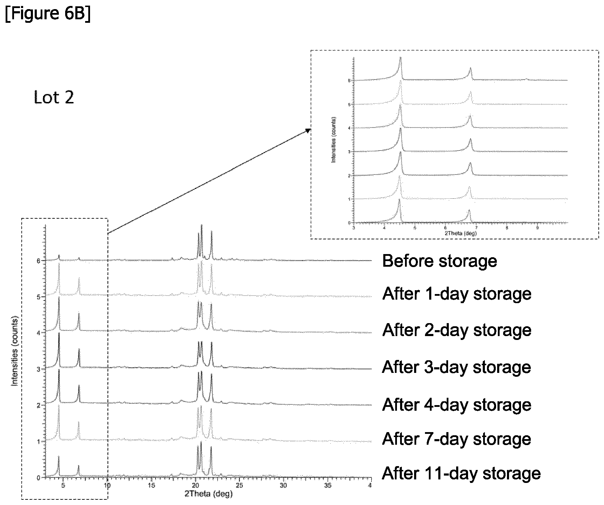

XRPD patterns of the samples from different lots (Lot 1, Lot 2) of sodium lauryl sulfate used in Example 1 stored up to day 11 are shown in Figure 6. In Lot 2, peaks of a sodium lauryl sulfate-dodecanol complex were not detected in the samples stored up to day 11; whereas, in Lot 1, peaks of a sodium lauryl sulfate-dodecanol complex were observed in samples stored for 3 days or more.

Sulfate ions generated by decomposition of sodium lauryl sulfate in the samples of Lot 1 and Lot 2 after 4-day storage were quantified by ion chromatography shown below.

As a result, the sulfate ion concentration in a sample solution prepared by using sodium lauryl sulfate (Lot 2) was 1 mg/L or less; whereas, that of sodium lauryl sulfate (Lot 1) was 24.1 mg/L. From the results, it was found that decomposition of sodium lauryl sulfate (Lot 1) in the samples after 4-day storage was proceeding.

Method for measuring sulfate ion by ion chromatography:
Sodium lauryl sulfate (about 5 mg) was weighed and water was added up to 50 mL. The solution was irradiated with ultrasonic wave for 15 minutes and filtered by a membrane filter (DISMIC-25HP PTFE 0.45 µm HYDROPHILIC, manufactured by ADVANTEC). The filtrate was passed through a solid-phase extraction cartridge (Dionex OnGuard II RP, manufactured by ThermoFisher Scientific) washed and the eluate was used as a sample solution. Separately, solutions having a sulfate ion concentration of 1 mg/L, 2 mg/L, 3 mg/L, 4 mg/L and 5 mg/L were prepared by use of the sulfate ion standard solution (1 g/L, manufactured by Wako Pure Chemical Industries Ltd.) for ion chromatography and used as the standard solutions. Experiments were carried out by using ion chromatography, Compact IC Flex (manufactured by Metrohm), in the following analysis conditions.

Column: Metrosep A Supp 7-250/4.0 (manufactured by Metrohm)
Guard column: Metrosep A Supp 4/5 S-Guard/4.0 (manufactured by Metrohm)
Suppressor: MSM,MCS (manufactured by Metrohm)
Eluate: 5 mM Na₂CO₃ solution
Cleaning liquid: purified water
Flow rate: 0.8 mL/min
Sample temperature: 20°C
Column temperature: 45°C
Injection volume: 10 ml
Measurement time: 24 min.

The concentration of sulfate ion in a sample solution was calculated based on the calibration curve prepared by using the standard solutions.

### Example 5: Study on pretreatment method for quality evaluation of pharmaceutical formulation (temperature)

From Example 3, it was found that impurity is present depending on the lot of sodium lauryl sulfate. Then, an ALECENSA capsule (150 mg) formulation (Lot A) was produced by using sodium lauryl sulfate (Lot 1), and an ALECENSA capsule (150 mg) formulation (Lot B) was produced by using sodium lauryl sulfate (Lot 3) (production date: 2015.10.02), which was produced by the same manufacturer at a different day, in accordance with Example 1 of Japanese Patent No. 5859712, and stored in different storage conditions. Whether an impurity, i.e., a complex, is present or not was checked.

Formulations (Lot A and Lot B) were placed in glass vials and stored in an airtight system at temperature conditions 5A (60°C), 5B (70°C) and 5C (80°C). Samples stored for 4 days, 7 days, 10 days and 14 days in condition 5A, and samples stored for 2 days and 3 days in condition 5B, and samples stored for one day in condition 5C were subjected to X-ray powder diffraction performed in the conditions shown in Example 1.

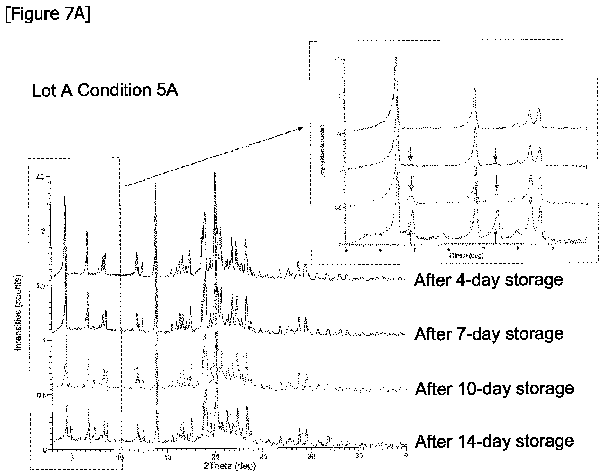

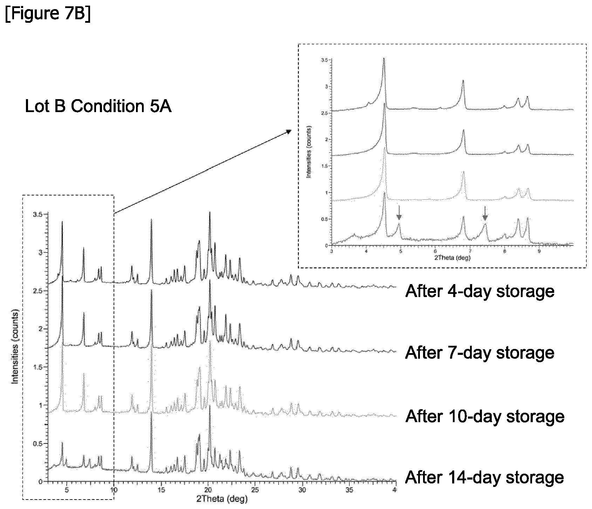

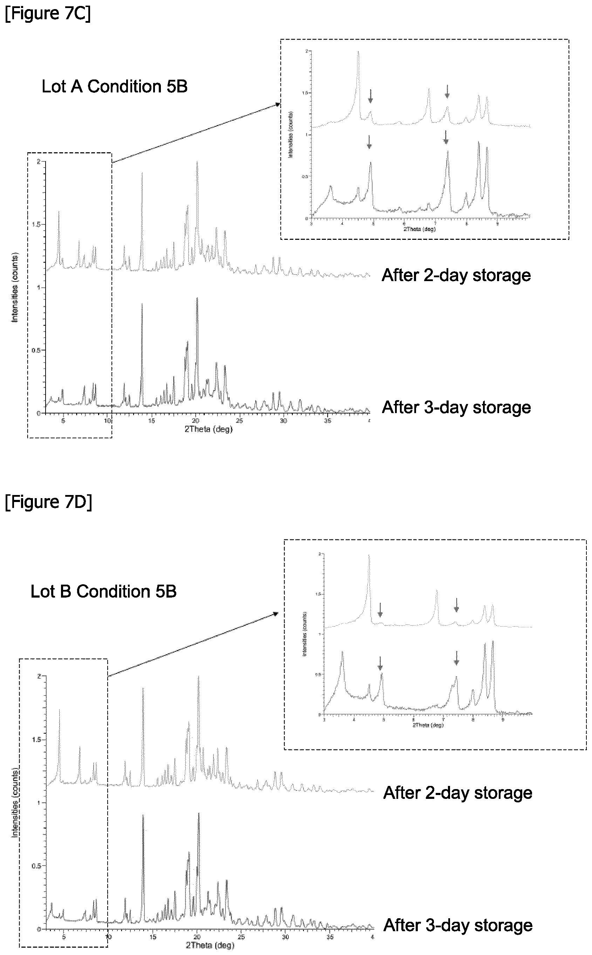

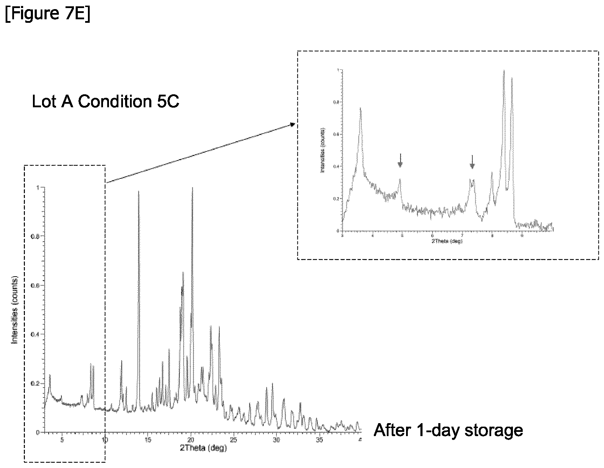

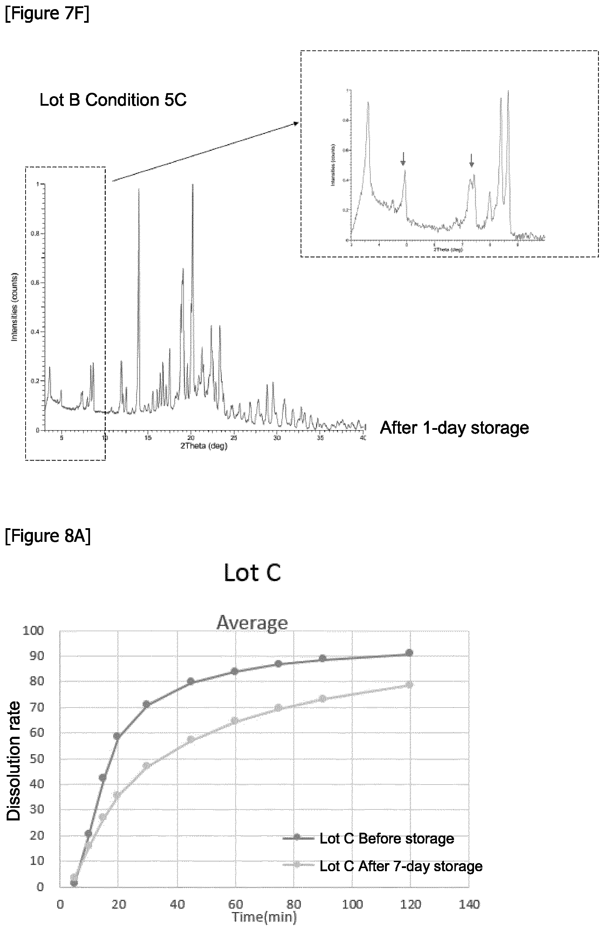

X-ray powder diffraction patterns of formulations (Lot A and Lot B) stored in conditions 5A, 5B and 5C are shown in Figure 7. Under condition 5A, peaks of a sodium lauryl sulfate-dodecanol complex were not detected in the formulation (Lot B) in the samples stored up to day 10; whereas peaks were detected in the formulation (Lot A) in the sample stored for 7 days.

In XRPD patterns of formulations (Lot A and Lot B) in individual temperature conditions, the storage periods at which the peak of a sodium lauryl sulfate-dodecanol complex was first detected are shown below.

### Condition 5A

Lot A: after 7-day storage
Lot B: after 14-day storage

### Condition 5B

Lot A: after 2-day storage
Lot B: after 2-day storage

### Condition 5C

Lot A: after 1-day storage
Lot B: after 1-day storage

From the results, condition 5A (60°C) was determined as the condition at which stability of formulations (Lot A and Lot B) can be evaluated and used for quality evaluation of formulations.

### Example 6: Effect of impurity of sodium lauryl sulfate on formulation

To examine the effect of impurity of sodium lauryl sulfate on formulation, an ALECENSA capsule (150 mg) formulation (Lot C) was produced by using sodium lauryl sulfate (Lot 1) of Example 3 and an ALECENSA capsule (150 mg) formulation (Lot D) was produced by using sodium lauryl sulfate (Lot 4) (production date: 2016.06.28), which was produced by the same manufacturer at a different day, in the same manner as in Example 5. The dissolution rates of individual formulations were checked.

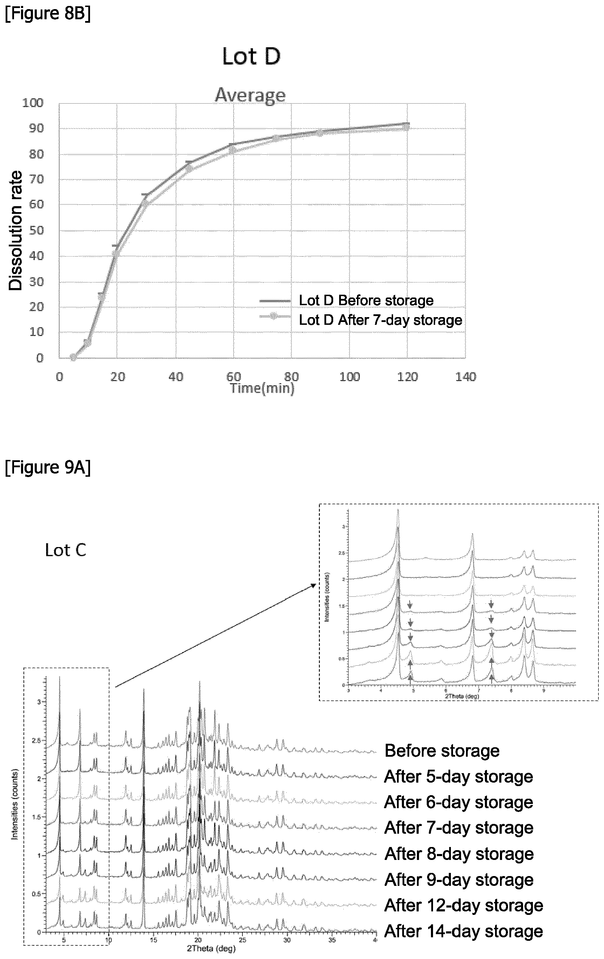

The formulation (Lot C) and formulation (Lot D) were placed in glass vials and stored in an airtight system at a temperature of 60°C. Samples before storage and samples stored for 7 days were subjected to a dissolution test performed in the following conditions. As a result, the dissolution profile of the formulation (Lot D) was the same as that of the formulation before storage; whereas, the dissolution rates of the formulation (Lot C) remarkably decreased from that of the formulation before storage (Figure 8).

### Method of dissolution test:

Using Solution 1 (900 mL) containing 7% of polyoxyethylene (10) octyl phenyl ether defined in the Japanese Pharmacopoeia dissolution test, as a test solution, tests were carried out in accordance with the paddle method defined in the dissolution test of the Japanese Pharmacopoeia, at 37°C and 100 rotations per minute.

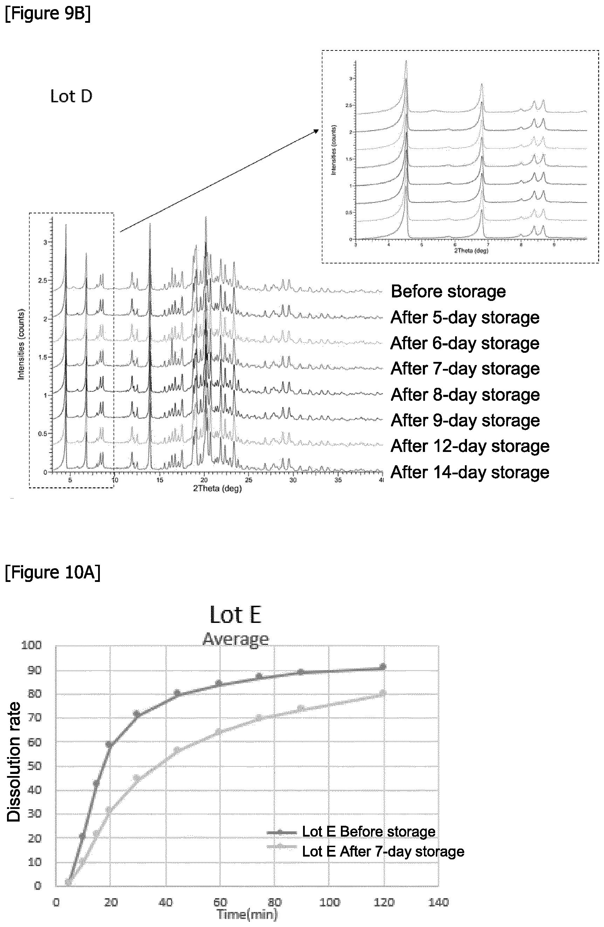

A sample before storage and samples stored for 5 days, 6 days, 7 days, 8 days, 9 days, 12 days and 14 days were subjected to X-ray powder diffraction performed in the conditions of Example 1. As a result, in formulations (Lot D), peaks of a sodium lauryl sulfate-dodecanol complex werre not detected until day 14; whereas in the formulations (Lot C), the peaks were detected in the sample stored for 7 days or more (Figure 9).

The sample stored for 7 days or more was subjected to quantification of dodecanol by GC/MS in accordance with the method shown below.

Method for measuring dodecanol by GC/MS:
The contents of 3 capsules of each of the formulations were taken out and mixed. The contents (50 mg) of the capsules were weighed and hexane was added up to 25 mL. The resultant mixtures were irradiated with ultrasonic wave for 30 minutes, transferred to centrifuge tubes and centrifuged. The supernatants were used as sample solutions. Separately, solutions having a dodecanol concentration of 1 g/L, 5 g/L, 10 g/L, 50 g/L and 100 g/L were prepared by using 1-dodecanol (manufactured by Wako Pure Chemical Industries Ltd.), and used as the standard solutions. Experiment was carried out by using GC/MS, 7850A (manufactured by Agilent) in the following analysis conditions.
Column: HP-5MS UI, 0.25 mm ID × 30 m, 0.25 µm (manufactured by Agilent)
Injection volume: 1.0 µL
Split ratio: 1: 10
Column temperature: 40°C (1 min)-> (20°C/min)-> 180°C-> (5°C/min)-> 190°C-> (50°C/min)-> 300°C (4 min)
Injector temperature: 300°C
Interface temperature: 30°C
Carrier gas: He
Flow rate: 1 mL/min
Ion voltage: 70 eV
Ionization mode: ESI-
Measurement mode: SIM
Monitor ion (m/z): 83.0

As a result, the dodecanol concentration in the formulation (Lot D) was a detection limit or less; whereas, the concentration of dodecanol/sodium lauryl sulfate (mol/mol%) in the formulation (Lot C) was 6.2%.

From the above, it was found that, in the formulation (Lot D), which was produced by using sodium lauryl sulfate (Lot 4), sodium lauryl sulfate is not decomposed and dissolution behavior thereof does not change by storage at 60°C for 7 days; whereas, in the formulation (Lot C), which was produced by using sodium lauryl sulfate (Lot 1), sodium lauryl sulfate is decomposed and dissolution rate thereof decreases by storage at 60°C for 7 days. Accordingly, it was found that if sodium lauryl sulfate having an appropriate quality is sorted out to produce a formulation, a formulation having a higher quality can be produced. More specifically, in order to sort out sodium lauryl sulfate (e.g., Lot 2) not easily decomposed and to eliminate sodium lauryl sulfate (e.g., Lot 1) easily decomposed, it was found that a method, in which a sample is stored for 4 days in accelerated condition including a temperature of 70°C and a relative humidity of about 10% and sulfate ion concentration is measured by ion chromatography, is effective in sorting a pharmaceutical raw material for a formulation for use in manufacturing a formulation having more excellent stability. Accordingly, sodium lauryl sulfate having a sulfate ion concentration of 1 mg/L or less, which is measured by the method, can be sorted out as a pharmaceutical raw material for a formulation that can produce a pharmaceutical formulation excellent in stability.

### Example 7: Setting of condition (temperature) for storing sodium lauryl sulfate

Sodium lauryl sulfate is usually stored at room temperature; however, there are possible cases, e.g., transportation, where it is stored at a higher temperature than room temperature. In consideration of high-temperature storage condition, a storage condition (temperature) was set up. Sodium lauryl sulfate (Lot 5) (production date: October 13, 2016 10) was wrapped with a plastic bag (primary packaging) and then with an aluminum bag (secondary packaging) and stored in the temperature conditions and periods shown in Table 1. Thereafter, a sample of about 250 mg of the sodium lauryl sulfate (Lot 5) was placed in a glass vial and stored in an open system at a temperature of 70°C, and a relative humidity of about 10% (a saturated aqueous solution of LiCI) for 4 days. After storage, sulfate ions generated by decomposition of sodium lauryl sulfate in the sample were quantified by ion chromatography shown in Example 4.

**[Table 1]**

| Temperature | 30°C | 40°C | 50°C | 60°C |
|---|---|---|---|---|
| Condition 1 | | 45 days | 15 hours | |
| Condition 2 | | 45 days | 24 hours | |
| Condition 3 | | 45 days | 48 hours | |
| Condition 4 | | 45 days | | 15 hours |
| Condition 5 | | 45 days | | 24 hours |
| Condition 6 | | 45 days | | 48 hours |
| Condition 7 | 45 days | | 15 hours | |
| Condition 8 | 45 days | | 24 hours | |
| Condition 9 | 45 days | | 48 hours | |
| Condition 10 | 45 days | | | 15 hours |
| Condition 11 | 45 days | | | 24 hours |
| Condition 12 | 45 days | | | 48 hours |
| Condition 13 | 45 days | | | |
| Condition 14 | 45 days | | | |
| Condition 15 | 45 days | 15 hours | | |
| Condition 16 | 45 days | 24 hours | | |
| Condition 17 | 45 days | 48 hours | | |

As a result, sulfate ion concentrations detected by ion chromatography were 1 mg/L or less in all conditions. Accordingly, the most severe condition, Condition 6 (at 40°C for 45 days and at 60°C for 48 hours), was set as the upper limit of the storage condition. The Lot of sodium lauryl sulfate which was stored not beyond this upper limit was determined that it was usefule for manufacturing a formulation having more excellent stability.

### Example 8: Relationship between impurity of sodium lauryl sulfate and quality (dissolution) of formulation

Using a plurality of formulations different in Lot, the relationship between the amount of dodecanol (an impurity of sodium lauryl sulfate) detected in a formulation and the dissolution of the formulation was examined. The formulations of Lot E (production date: 2016.08.24), Lot F (production date: 2016.08.29), Lot G (production date: 2016.08.24), Lot H (production date: 2016.10.19), Lot I (production date: 2016.03.29), Lot J (production date: 2016.03.24) and Lot K (production date: 2015.07.07) were individually placed in glass vials and stored in an airtight system at a temperature of 60°C. Samples before storage and samples stored for 7 days were subjected to a dissolution test. The samples stored for 7 days were subjected quantification of dodecanol by GC/MS. The dissolution test and quantification of dodecanol by GC/MS were carried out in accordance with the methods described in Example 6.

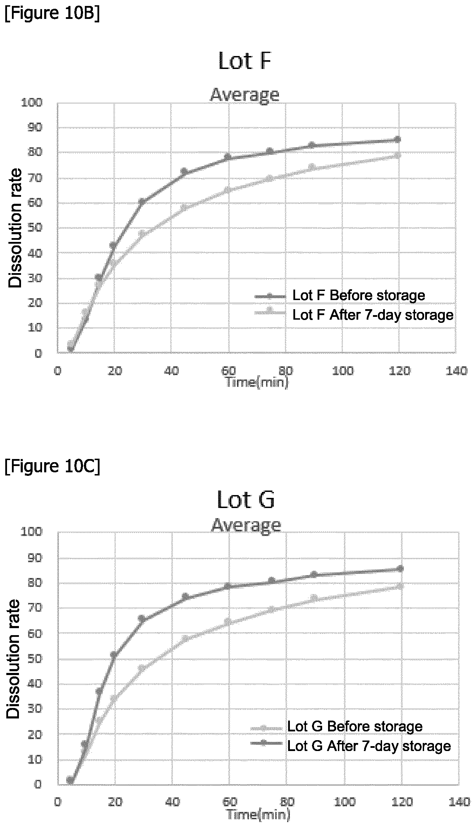

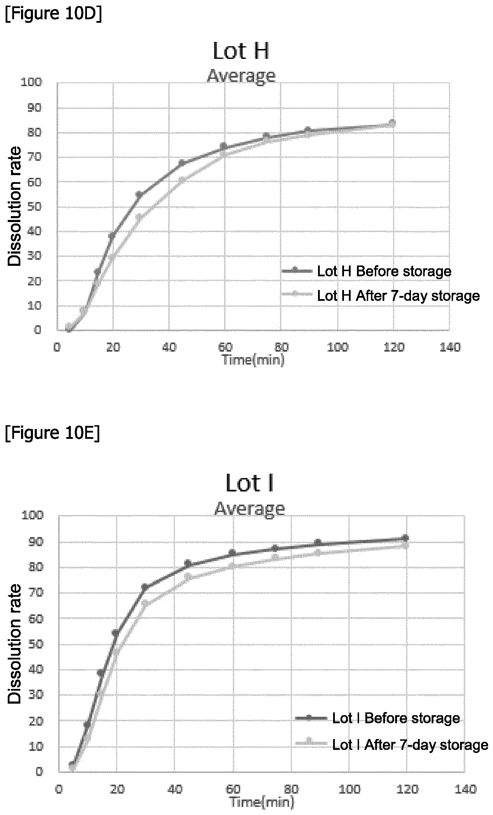

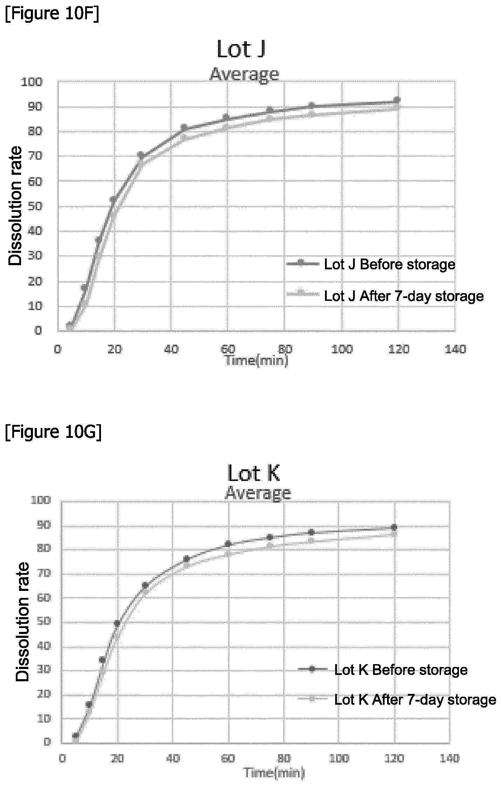

As a result of the dissolution test, the dissolution profiles of Lot H, Lot I, Lot J and Lot K were the same as that before storage; whereas, the dissolution rates of Lot E, Lot F and Lot G remarkably decreased from that before storage (Figure 10A to G). As a result of quantification of dodecanol in the samples after storage, the ratios of dodecanol/sodium lauryl sulfate (mol/mol%) in Lot H, Lot I, Lot J and Lot K were 2% or less; whereas, those of Lot E, Lot F and Lot G were all beyond 2% (Table 2).

**[Table 2]**

| Formulation lot | Dodecanol/sodium lauryl sulfate (mol/mol%) in formulation after storage at 60°C for 7 days |
|---|---|
| Lot E | 5.0% |
| Lot F | 3.0% |
| Lot G | 2.3% |
| Lot H | 0.4% |
| Lot I | 0.8% |
| Lot J | 0.5% |
| Lot K | Below quantification limit |

Based on the decomposition mechanism of sodium lauryl sulfate, it is considered that dodecanol/sodium lauryl sulfate (mol/mol%) obtained by decomposition is equal to sulfate ion/sodium lauryl sulfate (mol/mol%). Accordingly, from the results, it was found that if the ratio of dodecanol/sodium lauryl sulfate (mol/mol%) or sulfate ion/sodium lauryl sulfate (mol/mol%) detected is beyond 2% in a formulation stored in an airtight system at a temperature of 60°C for 7 days, dissolution of the resultant formulation decreases and the quality of the formulation is affected.

### Example 9: Confirmation of decomposition-kinetic properties of sodium lauryl sulfate in formulation

In Example 6, it was confirmed that the decomposition rate of sodium lauryl sulfate in a formulation varies depending on the formulation lot. The decomposition-kinetic properties were confirmed herein. Formulations (Lot A and Lot B) were stored in an airtight system at a temperature of 60°C. Samples of the formulation (Lot A) before storage, samples stored for 4 days, 7 days, 8 days, 9 days, 12 days and 14 days; and samples of the formulation (Lot B) stored for 4 days, 7 days, 8 days, 9 days and 12 days were subjected to quantification of dodecanol by GC/MS as described in Example 6.

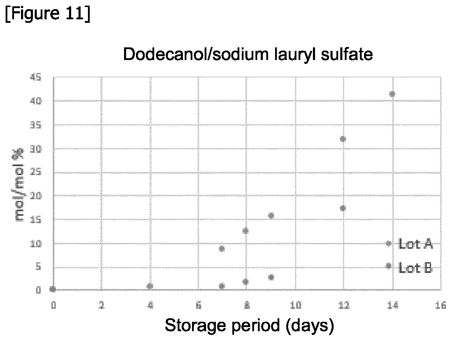

The results are shown in Figure 11. It was confirmed that the formulations (Lot A and Lot B) differ in period until dodecanol is detected, and that the amount of dodecanol once produced increases at an accelerated rate.

These conceivably reflect that sulfuric acid is generated by decomposition of sodium lauryl sulfate of a formulation and serves as a catalyst to promote decomposition, with the result that sodium lauryl sulfate is decomposed at an accelerated rate.

### Industrial Applicability

The present invention is used for sorting of a pharmaceutical raw material for a formulation, i.e., sodium lauryl sulfate, for use in manufacturing pharmaceuticals or sorting, quality evaluation and production of a composition and pharmaceutical containing sodium lauryl sulfate.

## Claims

1. A method for sorting a pharmaceutical raw material for a formulation consisting essentially of sodium lauryl sulfate, the method comprising detecting an impurity after pretreating the pharmaceutical raw material for a formulation under an accelerated condition.

2. A method for evaluating the quality of a pharmaceutical formulation containing a compound represented by formula (I) 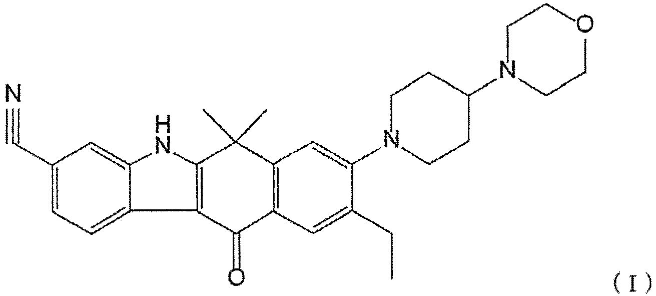 or a salt thereof and a pharmaceutical raw material for a formulation consisting essentially of sodium lauryl sulfate, the method comprising:
a) selecting a pharmaceutical formulation containing a sorted pharmaceutical raw material for a formulation, wherein the sorted pharmaceutical raw material for a formulation is the pharmaceutical raw material for a formulation which was sorted out by detecting an impurity after pretreating the pharmaceutical raw material for a formulation under the accelerated condition; and
b) skipping impurity measurement in the pharmaceutical formulation selected in step a.

3. The method according to claim 1 or 2, wherein the accelerated condition is a temperature condition of 65 to 75°C.

4. The method according to any one of claims 1 to 3, wherein the accelerated condition is a humidity condition of less than 79%RH.

5. The method according to any one of claims 1 to 4, wherein the accelerated condition is a humidity condition of about 10%RH or less.

6. The method according to any one of claims 1 to 5, wherein the accelerated condition includes a temperature condition of about 70°C and a humidity condition of about 10%RH or less.

7. The method according to any one of claims 1 to 6, wherein the impurity is a sulfate ion, dodecanol or a sodium lauryl sulfate-dodecanol complex.

8. The method according to any one of claims 1 to 7, wherein the impurity is detected by X-ray powder diffraction, ion exchange chromatography, gas chromatography or quantitative NMR.

9. The method according to any one of claims 1 to 8, wherein the impurity is a sulfate ion, and the impurity is detected by ion exchange chromatography.

10. The method according to any one of claims 1 to 9, wherein the pretreatment is performed under the accelerated condition for 2 days or more.

11. The method according to any one of claims 1 to 10, wherein the pretreatment is performed under the accelerated condition for 4 days.

12. A method for sorting a pharmaceutical raw material for a formulation consisting essentially of sodium lauryl sulfate, the method comprising:
a) sorting a lot or packaging unit of the pharmaceutical raw material for a formulation stored in a predetermined condition; and
b) skipping detection of an impurity in the pharmaceutical raw material for a formulation sorted out in step a.

13. The sorting method according to claim 12, wherein the condition includes 30°C to 40°C for 47 days or less.

14. The sorting method according to claim 12, wherein the condition includes 30°C to 40°C for 47 days or less, and if the temperature exceeds 40°C, a cumulative sum of time at which the temperature is beyond 40°C to 60°C or less falls within 48 hours.

15. The sorting method according to any one of claims 12 to 14, wherein the condition includes a storage condition during transportation.

16. A method for evaluating the quality of a pharmaceutical formulation containing sodium lauryl sulfate, the method comprising detecting an impurity after pretreating the pharmaceutical formulation containing sodium lauryl sulfate under an accelerated condition.

17. The quality evaluation method according to claim 16, wherein the pharmaceutical formulation contains a compound represented by formula (I) 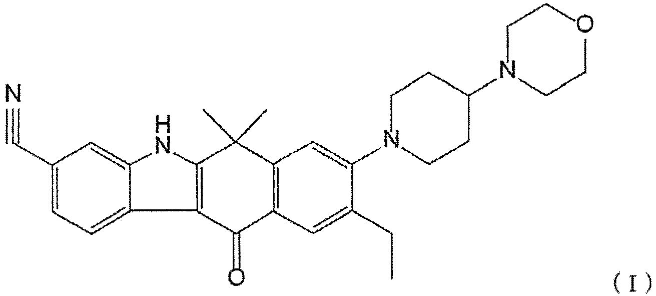 or a salt thereof.

18. The quality evaluation method according to claim 16 or 17, wherein the accelerated condition is a temperature condition of 55 to 65°C.

19. The quality evaluation method according to any one of claims 16 to 18, wherein the accelerated condition is a temperature condition of about 60°C.

20. The quality evaluation method according to any one of claims 16 to 19, wherein the impurity is a sulfate ion, dodecanol or a sodium lauryl sulfate-dodecanol complex.

21. The quality evaluation method according to any one of claims 16 to 20, wherein the impurity is detected by X-ray powder diffraction, ion exchange chromatography, gas chromatography or quantitative NMR.

22. The quality evaluation method according to any one of claims 16 to 21, wherein the impurity is dodecanol, and the impurity is detected by gas chromatography.

23. The quality evaluation method according to any one of claims 16 to 21, wherein the impurity is a sulfate ion, and the impurity is detected by ion exchange chromatography.

24. The quality evaluation method according to any one of claims 16 to 23, wherein the pretreatment is performed under the accelerated condition for 2 days or more.

25. The quality evaluation method according to any one of claims 16 to 24, wherein the pretreatment is performed under the accelerated condition for 7 days.

26. The quality evaluation method according to any one of claims 16 to 25, wherein a pharmaceutical formulation in which 2% or less of dodecanol/sodium lauryl sulfate (mol/mol%) or a sulfate ion/sodium lauryl sulfate (mol/mol%) as the impurity is present, is sorted out.

27. A method for manufacturing a pharmaceutical formulation containing sodium lauryl sulfate, the method comprising:
a) pretreating sodium lauryl sulfate of a pharmaceutical raw material for a formulation under an accelerated condition;
b) detecting an impurity after the pretreatment of step a to sort out a pharmaceutical raw material for a formulation in which the content of a sulfate ion as the impurity is 1 mg/L or less; and
c) manufacturing a pharmaceutical formulation by using the pharmaceutical raw material for a formulation sorted out by step b.

28. The manufacturing method according to claim 27, wherein the pharmaceutical formulation contains a compound represented by formula (I) 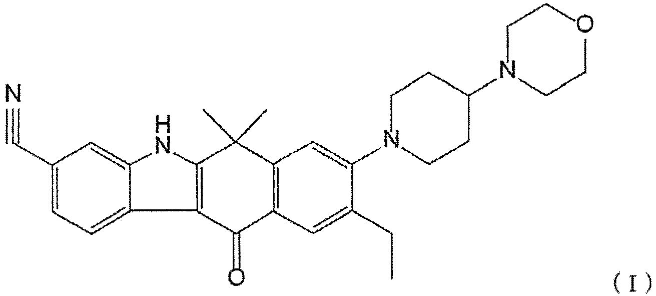 or a salt thereof.

29. A method for manufacturing a pharmaceutical formulation containing a compound represented by formula (I) 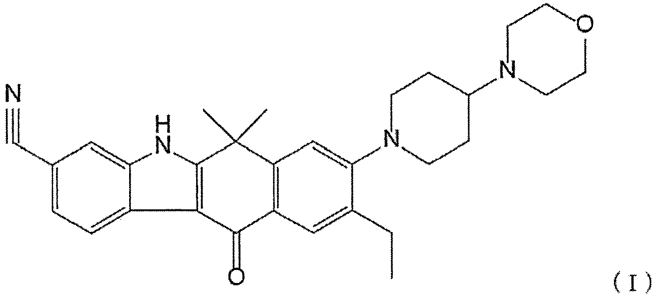 or a salt thereof and sodium lauryl sulfate, the method comprising:
a) manufacturing the pharmaceutical formulation by using a pharmaceutical raw material for a formulation consisting essentially of sodium lauryl sulfate;
b) pretreating the pharmaceutical formulation produced in step a; and
c) detecting an impurity after the pretreatment of step b to sort out a pharmaceutical formulation in which the content of dodecanol/sodium lauryl sulfate (mol/mol%) or sulfate ion/sodium lauryl sulfate (mol/mol%) as the impurity is 2% or less.

30. A pharmaceutical composition containing a compound represented by formula (I) 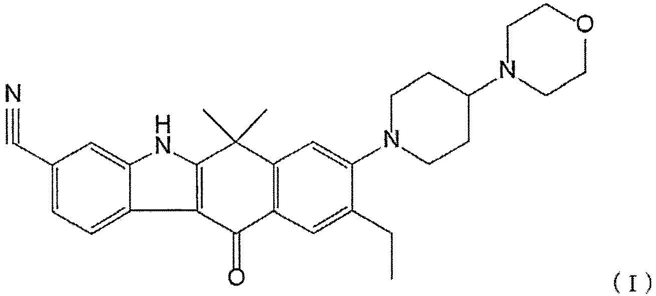 or a salt thereof and sodium lauryl sulfate, wherein dodecanol/sodium lauryl sulfate or sulfate ion/sodium lauryl sulfate is contained in a ratio such that it causes no effect on dissolution in a pharmaceutical formulation containing the composition when the formulation is measured after a pretreatment under an accelerated condition.

31. The composition according to claim 30, wherein the content of dodecanol/sodium lauryl sulfate (mol/mol%) or sulfate ion/sodium lauryl sulfate (mol/mol%) in the composition, as measured by X-ray powder diffraction, ion exchange chromatography, gas chromatography or quantitative NMR is 2% or less.

32. A pharmaceutical formulation containing a compound represented by formula (I) 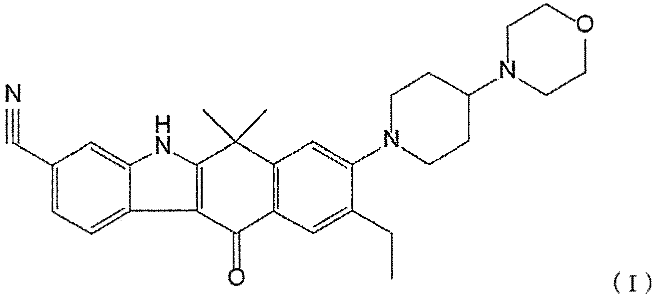 or a salt thereof and sodium lauryl sulfate, wherein dodecanol/sodium lauryl sulfate or sulfate ion/sodium lauryl sulfate is contained in a ratio such that it causes no effect on dissolution behavior in the formulation when the composition is measured after a pretreatment under an accelerated condition.

33. The formulation according to claim 32, wherein the ratio of dodecanol/sodium lauryl sulfate (mol/mol%) or sulfate ion/sodium lauryl sulfate (mol/mol%) in the formulation is 2% or less, as measured by X-ray powder diffraction, ion exchange chromatography, gas chromatography or quantitative NMR.

34. A pharmaceutical formulation containing sodium lauryl sulfate, wherein the ratio of dodecanol/sodium lauryl sulfate or sulfate ion/sodium lauryl sulfate in the formulation such that it causes no effect on dissolution behavior when the formulation is measured after a pretreatment under an accelerated condition.

35. A pharmaceutical raw material for a formulation consisting essentially of sodium lauryl sulfate, wherein the content of the sulfate ion in the material is 1 mg/L or less when the pharmaceutical raw material for a formulation is pretreated under an accelerated condition and thereafter measured by ion exchange chromatography.
